(19) Europäisches Patentamt
European Patent Office
Office européen des brevets

(11) Publication number: **0 064 033 A1**

(12) **EUROPEAN PATENT APPLICATION**

(21) Application number: **82810175.8**

(22) Date of filing: **27.04.82**

(51) Int. Cl.³: **C 07 D 498/04**, C 07 D 513/04, C 07 D 487/04, C 07 F 7/18, A 61 K 31/55, A 61 K 31/505, A 61 K 31/645 // (C07D498/04, 263/00, 239/00), (C07D498/04, 265/00, 239/00)

(30) Priority: **28.04.81 JP 64738/81**

(43) Date of publication of application: **03.11.82 Bulletin 82/44**

(84) Designated Contracting States: **CH DE FR IT LI**

(71) Applicant: **Shionogi & Co., Ltd., 12, 3-chome Dosho-machi Higashi-ku, Osaka (JP)**

(72) Inventor: **Kamata, Susumu, 1-8-14, Hikarigaoka, Takarazuka-shi Hyogo Prefecture (JP)**
Inventor: **Nagata, Wataru, 6-10, Kawahigashi-cho, Nishinomiya-shi Hyogo Prefecture (JP)**

(74) Representative: **Bovard, Fritz Albert et al, Bovard & Co Optingenstrasse 16, CH-3000 Bern 25 (CH)**

(54) **5-Fluorouracil derivatives.**

(57) Novel 5-fluorouracil derivatives of the following general formula

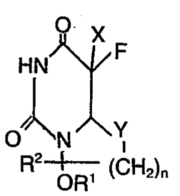

(wherein $R^1$ is hydrogen, $C_1$-$C_5$alkyl, $C_6$-$C_{10}$aryl, $C_7$-$C_{10}$aralkyl, $C_1$-$C_{12}$alkanoyl, $C_2$-$C_6$alkoxycarbonyl, $C_1$-$C_5$alkanoyloxymethyl, carbamoyl or tri-$C_1$-$C_5$alkylsilyl;
$R^2$ is hydrogen, $C_1$-$C_5$alkyl, $C_6$-$C_{10}$aryl or $C_7$-$C_{10}$aralkyl;
X is hydrogen, halogen or $C_2$-$C_6$alkoxycarbonyl;
Y is O, NR' (R' is hydrogen or $C_1$-$C_5$alkyl), S, SO or $SO_2$; and
n is an integer of 1-3),
which are orally administrable anti-tumor agents.

EP 0 064 033 A1

# 5-Fluorouracil Derivatives

The invention relates to novel 5-fluorouracil derivatives, which are orally applicable anti-tumor agents.

5-Fluorouracil (hereinafter abbreviated to as 5FU) has widely been used in treatment of malignant tumors as antimetabolic anti-tumor agents. The anti-tumor activity of 5FU is very strong, but it has many disadvantages such as the restricted scope of application, limited dosage, forbidden continuous administration over long periods and difficult oral application because of its strong toxity and adverse reaction of frequent occurrence. As a modified 5FU derivative, FT-207 [1-(2-tetrahydrofuryl)5-fluorouracil] [Ftorafur® (Taiho Yakuhin); Jap. Pat. Unexam. Pub. Nos. 50-50383; 50-50384; 50-64281; 51-14682; 53-84981] which is a less toxic, orally and continuously administrable derivative, the activity of which is, however, insufficient is now commercially available. Other 5FU derivatives under clinical evaluation, for example, HCFU [1-n-hexylcarbamoyl-5-fluorouracil] [Mitsui Seiyaku; Jap. Pat. Unexam. Pub. No. 50-148365] are known.

The present invention relates to novel 5-fluorouracil derivatives. More particularly, it relates to orally applicable novel anti-tumor compounds, 5-fluorouracil derivatives, which are represented by the following general formula (I).

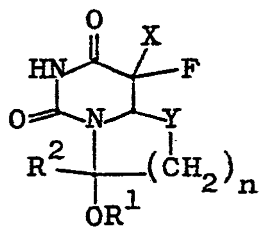

(I)

[wherein $R^1$ is hydrogen, $C_1$-$C_5$alkyl, $C_6$-$C_{10}$aryl, $C_7$-$C_{10}$ aralkyl, $C_1$-$C_{12}$alkanoyl, $C_2$-$C_6$alkoxycarbonyl, $C_1$-$C_5$alkanoyloxymethyl, carbamoyl or tri-$C_1$-$C_5$alkylsilyl ;

$R^2$ is hydrogen, $C_1$-$C_5$alkyl, $C_6$-$C_{10}$aryl or $C_7$-$C_{10}$ aralkyl ;

X is hydrogen, halogen or $C_2$-$C_6$alkoxycarbonyl ;

Y is O, NR' (R' is hydrogen or $C_1$-$C_5$alkyl), S, SO or $SO_2$ ; and

n is an integer of 1-3]

The purpose of the present invention is to provide 5FU derivatives of pro-drug type in order to make varied application forms possible, particularly to provide orally administrable derivatives with less adverse reaction, and

to improve permeability of 5FU to the tumor cells.
The present invention offers the compounds of the above general formula (I) as 5FU derivatives which are suitable for the above purposes.

The objective compounds in the present invention are represented by the general formula (I) :

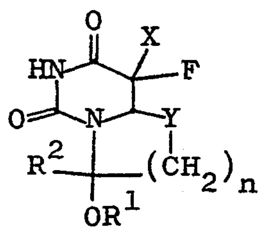

(I)

[wherein $R^1$ is hydrogen, $C_1$-$C_5$alkyl, $C_6$-$C_{10}$aryl, $C_7$-$C_{10}$ aralkyl, $C_1$-$C_{12}$alkanoyl, $C_2$-$C_6$alkoxycarbonyl, $C_1$-$C_5$alkanoyloxymethyl, carbamoyl or tri-$C_1$-$C_5$alkylsilyl ;

$R^2$ is hydrogen, $C_1$-$C_5$alkyl, $C_6$-$C_{10}$aryl or $C_7$-$C_{10}$ aralkyl ;

X is hydrogen, halogen or $C_2$-$C_6$alkoxycarbonyl ;

Y is O, NR' (R' is hydrogen or $C_1$-$C_5$alkyl), S, SO or $SO_2$ ; and

n is an integer of 1-3]

In the definition of the above general formula (I), $C_1$-$C_5$alkyl means a straight or branched chain lower alkyl of 1-5 carbon atoms, e.g., methyl, ethyl, propyl, isopropyl, butyl, isobutyl, sec-butyl, pentyl.

$C_6$-$C_{10}$Aryl means phenyl or naphthyl of 6-10 carbon atoms which may be substituted by lower alkyls (e.g. methyl, ethyl, propyl), lower alkoxys (e.g., methoxy, ethoxy, propoxy), halogen (e.g., fluoro, chloro, bromo), nitro and the like, including phenyl, p-toluyl, p-methoxyphenyl, 2,4-dimethoxyphenyl, p-chlrophenyl and p-nitrophenyl. $C_7$-$C_{10}$Aralkyl means benzyl or phenethyl of 7-10 carbon atoms which may be substituted, including benzyl, p-methoxybenzyl, p-chlorobenzyl, p-toluyl, phenethyl and (3,5-dimethylphenyl)ethyl. $C_1$-$C_{12}$Alkanoyl means an alkanoic acid residue of 1-12 carbon atoms derived from fatty acids, e.g., formyl, acetyl, propionyl, butyryl, valeryl, pivaloyl, octanoyl. $C_2$-$C_6$ Alkoxycarbonyl means a straight or branched chain lower alkoxycarbonyl of 2-6 carbon atoms, e.g., methoxycarbonyl, ethoxycarbonyl, propoxycarbonyl, isopropoxycarbonyl, t-butoxycarbonyl, pentoxycarbonyl. $C_1$-$C_5$Alkanoyloxymethyl means an oxymethyl substituted by an alkanoyl of 1-5 carbon atoms, e.g. acetyloxymethyl, propionyloxymethyl, butyryloxymethyl, pivaloyloxymethyl. Tri- $C_1$-$C_5$alkylsilyl means a group in which the silyl is substituted by 3 $C_1$-$C_5$alkyls mentioned above, e.g., trimethylsilyl, triethylsilyl, tripropylsilyl, tripentylsilyl, t-butyldimethylsilyl. Halogen includes chloro, bromo and iodo.

Representative of the objective compounds in the present invention are :

8α-Bromo-8β-fluoro-3α-hydroxy-2,3,6,7,8,8aα-hexahydro-5,7-dioxo-5H-oxazolo[3,2-c]pyrimidine,

8β-Fluoro-3α-hydroxy-2,3,6,7,8,8aα-hexahydro-5,7-dioxo-5H-oxazolo[3,2-c]pyrimidine,

3α-Acetoxy-8β-fluoro-2,3,6,7,8,8aα-hexahydro-5,7-dioxo-5H-oxazolo[3,2-c]pyrimidine,

8β-Fluoro-2,3,6,7,8,8aα-hexahydro-3α-octanoyloxy-5,7-dioxo-5H-oxazolo[3,2-c]pyrimidine,

8β-Fluoro-2,3,6,7,8aα-hexahydro-5,7-dioxo-3α-trimethylacetoxy-5H-oxazolo[3,2-c]pyrimidine,

3α-Acetoxy-8α-bromo-8β-fluoro-2,3,6,7,8,8aα-hexahydro-5,7-dioxo-5H-oxazolo[3,2-c]pyrimidine,

8α-Bromo-8β-fluoro-2,3,6,7,8,8aα-hexahydro-3α-octanoyloxy-5,7-dioxo-5H-oxazolo[3,2-c]pyrimidine,

8α-Bromo-8β-fluoro-2,3,6,7,8,8aα-hexahydro-5,7-dioxo-3α-trimethylacetoxy-5H-oxazolo[3,2-c]pyrimidine,

3α-(tert-Butyldimethylsilyloxy)-8α-bromo-8β-fluoro-2,3,6,7,8,8aα-hexahydro-5,7-dioxo-5H-oxazolo[3,2-c]pyrimidine,

3α-(tert-Butyldimethylsilyloxy)-8α-fluoro-2,3,6,7,8,8aα-hexahydro-5,7-dioxo-5H-oxazolo[3,2-c]-pyrimidine,

3α-(tert-Butyldimethylsilyloxy)-8β-fluoro-2,3,6,7,8,8aα-hexahydro-5,7-dioxo-5H-oxazolo[3,2-c]-pyrimidine,

8α-Fluoro-3α-hydroxy-2,3,6,7,8,8aα-hexahydro-5,7-dioxo-5H-oxazolo[3,2-c]pyrimidine,

3α-Acetoxy-8α-fluoro-2,3,6,7,8,8aα-hexahydro-5,7-dioxo-5H-oxazolo[3,2-c]pyrimidine,

8α-Fluoro-2,3,6,7,8,8aα-hexahydro-3α-octanoyloxy-5,7-dioxo-5H-oxazolo[3,2-c]pyrimidine,

8α-Fluoro-2,3,6,7,8,8aα-hexahydro-5,7-dioxo-3α-trimethylacetoxy-5H-oxazolo[3,2-c]pyrimidine,

9α-Bromo-9β-fluoro-3,4,7,8,9,9aα-hexahydro-4α-hydroxy-6,8-dioxo-2H,6H-[1,3]-oxazino[3,2-c]pyrimidine,

9β-Fluoro-6,8-dioxo-4α-hydroxy-3,4,7,8,9,9aα-hexahydro-2H,6H-[1,3]-oxazino[3,2-c]pyrimidine,

4α-(tert-Butyldimethylsilyloxy)-7-tert-butyldimethylsilyl-9α-bromo-9β-fluoro-3,4,7,8,9,9aα-hexahydro-6,8-dioxo-2H,6H-[1,3]-oxazino[3,2-c]pyrimidine,

4α-(tert-Butyldimethylsilyloxy)-9α-fluoro-3,4,7,8,9,9aα-hexahydro-6,8-dioxo-2H,6H-[1,3]-oxazino-[3,2-c]pyrimidine,

9α-Fluoro-3,4,7,8,9,9aα-hexahydro-4α-hydroxy-6,8-dioxo-2H,6H-[1,3]-oxazino[3,2-c]pyrimidine,

9α-Fluoro-3,4,7,8,9,9aα-hexahydro-4β-hydroxy-6,8-dioxo-2H,6H-[1,3]-oxazino[3,2-c]pyrimidine,

4α-Acetoxy-9α-bromo-9β-fluoro-3,4,7,8,9,9aα-hexahydro-6,8-dioxo-2H,6H-[1,3]-oxazino[3,2-c]pyrimidine,

4α-Acetoxy-9α-fluoro-3,4,7,8,9,9aα-hexahydro-6,8-dioxo-2H,6H-[1,3]-oxazino[3,2-c]pyrimidine,

4α-Acetoxy-9β-fluoro-3,4,7,8,9,9aα-hexahydro-6,8-dioxo-2H,6H-[1,3]-oxazino[3,2-c]pyrimidine,

9α-Bromo-9β-fluoro-3,4,7,8,9,9aα-hexahydro-4α-octanoyloxy-6,8-dioxo-2H,6H-[1,3]-oxazino[3,2-c]pyrimidine,

9α-Bromo-9β-fluoro-3,4,7,8,9,9aα-hexahydro-6,8-dioxo-4α-trimethylacetoxy-2H,6H-[1,3]-oxazino[3,2-c]-pyrimidine,

9α-Fluoro-3,4,7,8,9,9aα-hexahydro-6,8-dioxo-4α-trimethylacetoxy-2H,6H-[1,3]-oxazino[3,2-c]pyrimidine,

9α-Fluoro-3,4,7,8,9,9aα-hexahydro-4α-octanoyloxy-6,8-dioxo-2H,6H-[1,3]-oxazino[3,2-c]pyrimidine,

9β-Fluoro-3,4,7,8,9,9aα-hexahydro-4α-octanoyloxy-6,8-dioxo-2H,6H-[1,3]-oxazino[3,2-c]pyrimidine.

The objective compounds (I) in the present invention can be produced from the known compounds according to the process shown in the following reaction sequence.

(1) X=halogen, Y=O

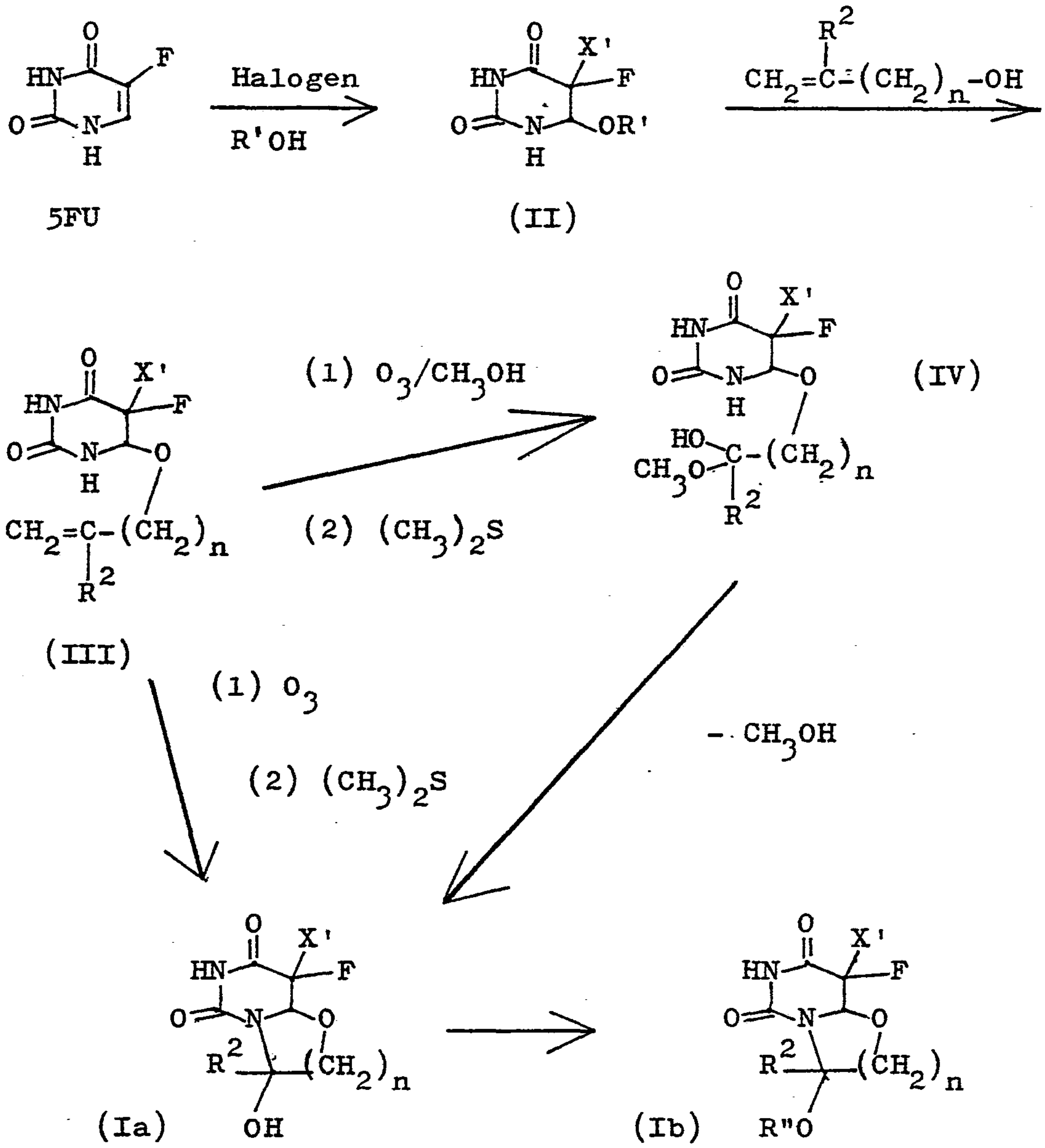


5FU (II) (III) (IV) (Ia) (Ib)

[wherein R' is hydrogen, lower alkvl or lower alkanoyl ; R" has the same significance as $R^1$ except for hydrogen ; X' is halogen ; $R^2$ and n have the same significance as mentioned above]

In the above reaction sequence, the conversion

of the starting compound, 5FU to the halogen adduct (II) is achieved by reacting 5FU with lower alkanols or with lower alkanoic acids and the corresponding acid anhydride in the presence of a halogen such as $Br_2$ wherein the lower alkanols or alkanoic acids are represented by the general formula R'OH, according to the known methods, for example, described in J. Med. Chem. 10, 47 (1967).

The conversion of the halogen adducts (II) to the derivatives (III) is achieved on reaction with unsaturated alcohols represented by the general formula $CH_2=C(R^2)-(CH_2)_n-OH$ in the presence of a catalytic amount of acid, for example, methanesulfonic acid. The reaction may be conducted in or without a solvent under heating at the refluxing temperature for about 1-10 hours. As the reaction solvents, aprotic solvents such as benzene, toluene, acetone, acetonitrile, tetrahydrofuran, dioxane and N,N-dimethylformamide and the like may preferably be employed. In some instances the reaction may preferably be conducted in a reaction vessel equipped with a water separator containing Molecular sieves.

Ozonolysis of the alcohol adduct (III) gives the compounds (Ia), one of the objective compounds in the present invention. The reaction may be conducted under the condition employed in the conventional ozonolysis. In addition of this process of producing the compounds (Ia) derectly from the compounds (III) by ozonolysis, the compounds (Ia) may

be prepared through the stable intermediates (IV) by ozonolysis followed by elimination of methanol accompanied by cyclization. The stable intermediate (IV) in ozonolysis can be obtained by carrying out the reation in the presence of methanol.

The acylation or alkylation of the compound (Ia) according to the known method gives the compound (Ib) in which $R^1$ is not hydrogen.

The compounds in which Y is not O but NR', S, SO and $SO_2$ can also be produced in the same way as in the compounds of Y being O.

(2) X=hydrogen, Y=O

(a)

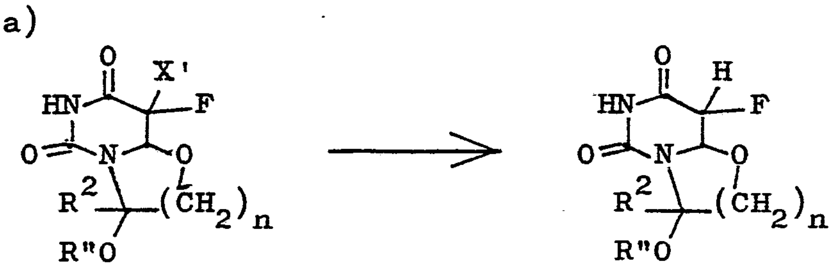

(Ib) (Ic)

[wherein R", X' and n have the same significance as mentioned above ]

In the general formula (I), the compounds in which $R^1$ is the same as in the above definition except for hydrogen, X is hydrogen and Y is O can be prepared from the compound (Ib) on reductive elimination of the halogen X'. As the reduction condition, hydrogenolysis with nickel, palladium, platinum or rhodium catalyst, or hydride reduction

with tri-n-butyltin hydride can be employed. For example, in case of hydrogenolysis with a palladium catalyst, the reaction may be carried out in the conditions of catalytic hydrogenation conducted in tetrahydrofuran, dioxane or a mixture of tetrahydrofuran-methanol in the presence of a weak base such as sodium acetate employing palladium-carbon as a catalyst. Alternatively, reduction with potassium hydrogensulfide (KSH) in an alcohol such as methanol and ethanol may also be employed.

(b)

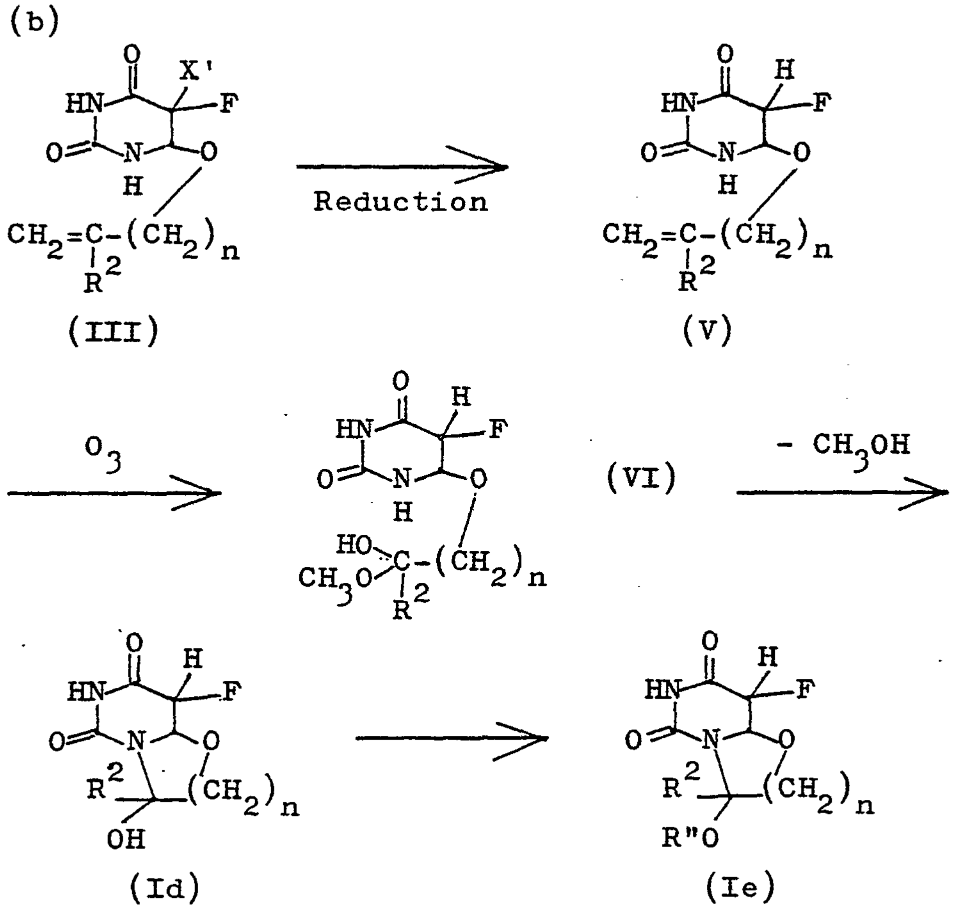


(III) (V) (VI) (Id) (Ie)

[wherein $R^2$, R", X' and n have the same significance as mentioned above]

The alcohol substituted derivatives (III) are reduced in the same manner as in the above step (a), and the subsequent reaction of the resulting compounds (V) may be carried out according to the reaction sequence via the above compounds (IV) to the compounds (Ia) and (Ib). The reaction in each step can be conducted in the same manner as mentioned above.

The reaction in the above steps (a) and (b) can also be applied in producing the compounds in which Y is not O but NR', S, SO and $SO_2$ in the general formula (I).

(3) X=alkoxycarbonyl, Y=O

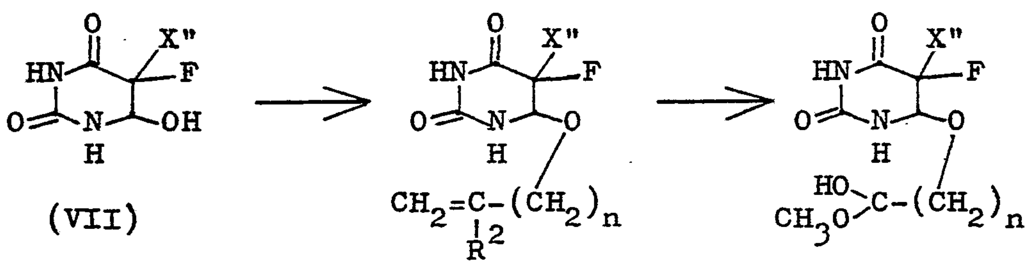

(VII) (VIII) (IX)

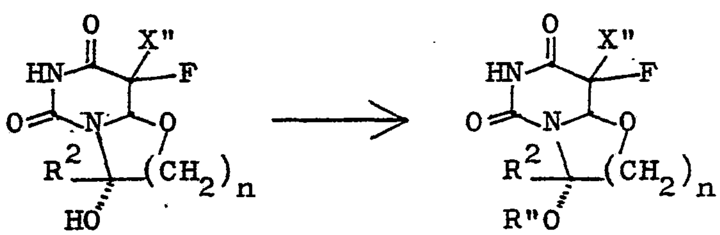

(If) (Ig)

[wherein X" is $C_2$-$C_6$alkoxycarbonyl, and $R^2$, R" and n have the same significance as mentioned above]

The reaction of the starting compounds (VII) to the alcohol substituted compounds (VIII) can be carried

out according to the substitution reaction of an unsaturated alcohol $CH_2=C(R^2)-(CH_2)_n-OH$ with the above compounds (II) to give (III). The reaction from the alcohol substituted compounds (VIII) to the compounds (IX), (If) and (Ig) can be achieved under the same condition as in the reaction from the above compounds (III) to (Ia) and (Ib). The starting compounds (VII) and the intermediate (VIII) are the known compounds, disclosed, for example,in Jap. Pat. Unexam. pub. No.55-102573.

The compounds in which Y is not O but NR', S, SO and $SO_2$ in the general formula (I) can be produced in the same procedure as mentioned above.

The objective compounds (I) (Ia-g) in the present invention are orally applicable and have a superior anti-tumor action. For example, the anti-tumor activity of the compounds (I) against leukemia L1210 in mice is as follows.

(Test Method)

Ascites cells ($10^5$ cell) of leukemia L1210 in mice are diluted with physiological saline and intraperitoneally inplanted in the $BDF_1$ female mice of 5 weeks age. 8-10 Mice are employed in a control group, and 6-7 mice are employed in a test group to which the test compounds are given. A prefixed amount of the test compounds is administered to the mice of the test group intraperitoneally

or orally successively for 5 days.

(Judgement of Effect)

From the average survival days in each test group and control group, the increase of lifespan (ILS) is calculated according to the following equation.

$$\text{ILS}(\%) = \frac{\begin{pmatrix}\text{Average survival}\\ \text{days in administered}\\ \text{group}\end{pmatrix} - \begin{pmatrix}\text{Average survival}\\ \text{days in control}\\ \text{group}\end{pmatrix}}{(\text{Average survival days in control group})} \times 100$$

From the dosage of maximum ILS value (maximum effective dose) and 30% ILS value (minimum effective dose), chemotherapeutic index (CI) is calculated according to the following equation. The higher value indicates the higher safety.

$$\text{CI} = \frac{\text{Maximum effective dose (mg/kg)}}{\text{Minimum effective dose (mg/kg)}}$$

(Compounds Tested)

A: 5FU

B: Ftorafur®

C: dl-8α-Fluoro-2,3,6,7,8,8aα-hexahydro-5,7-dioxo-3α-trimethylacetoxy-5H-oxazolo[3,2-c]pyrimidine

D: dl-8α-Fluoro-2,3,6,7,8,8aα-hexahydro-3α-octanoyloxy-5,7-dioxo-5H-oxazolo[3,2-c]pyrimidine

E: dl-3α-Acetoxy-8α-fluoro-2,3,6,7,8,8aα-hexahydro-5,7-dioxo-5H-oxazolo[3,2-c]pyrimidine

F: dl-9α-Fluoro-3,4,7,8,9,9aα-hexahydro-6,8-dioxo-4α-trimethylacetoxy-2H,6H-[1,3]-oxadino[3,2-c]pyrimidine

(Result)

Table 1 ILS value (%) of each compound administered intraperitoneally

| Dose (mg x day) | Compounds Tested | | | |
|---|---|---|---|---|
| | A | B | C | E |
| 4 x 5 | 20 | — | — | — |
| 20 x 5 | 50 | — | — | — |
| 40 x 5 | 84 | 0 | 7 | 14 |
| 100 x 5 | 13 | 26 | 13 | 18 |
| 200 x 5 | — | 36 | 32 | 28 |
| 400 x 5 | — | 4 | 37 | 58 |
| 600 x 5 | — | — | 39 | — |

Table 2 CI value of each compound administered intraperitoneally

| Compounds | A | B | C | E |
|---|---|---|---|---|
| Maximum Effective Dose (mg/kg) | 200 | 1000 | — | 2000 |
| Minimum Effective Dose (mg/kg) | 40 | 700 | — | 1000 |
| CI Value | 5.0 | 1.4 | — | 2 |

Table 3 ILS value (%) of each compound administered orally

| Dose (mg x day) | Compounds Tested | | | | | |
|---|---|---|---|---|---|---|
| | A | B | C | D | E | F |
| 20 x 5 | 22 | — | — | — | — | — |
| 40 x 5 | 39 | 1 | — | 8 | 1 | 9 |
| 60 x 5 | 56 | — | — | — | — | — |
| 100 x 5 | 35 | 19 | 21 | 22 | - 3 | 13 |
| 200 x 5 | — | 29 | 47 | 30 | 14 | 32 |
| 400 x 5 | — | 31 | 59 | 38 | 39 | 51 |
| 600 x 5 | — | - 9 | 107 | 51 | 46 | 57 |

Table 4 CI value of each compound administered orally

| Compounds | A | B | C | D | E | F |
|---|---|---|---|---|---|---|
| Maximum Effective Dose (mg/kg) | 300 | 2000 | 3000 | 3000 | 2400 | 3000 |
| Minimum Effective Dose (mg/kg) | 150 | 1000 | 1000 | 1000 | 1200 | 1000 |
| CI Value | 2.0 | 2.0 | 3.0 | 3.0 | 2.0 | 3.0 |

Obviously as shown in the above result, the compounds (I) in the present invention have a superior anti-tumor action and can be applied to human or animals as anti-tumor agents.

In addition, the compounds (I) are advantageous in view of easiness of increase or decrease of dosage, because the effective range of the dose is relatively wide.

The compounds (I) in the present invention can be administered to human or animals orally or parenterally. For example, the compounds (I) can be administered intravenously, intramuscularly, or subcutaneously, etc. as solutions or suspensions in a proper solvent for injection (e.g., distilled water for injection, ethanol, glycerol, propylene glycol, olive oil, peanut oil). In preparations for injection, the compounds (I) can be kept in ampouls in a form of solutions or suspensions, and more preferably preserved in ampoules or vials in a form of crystals, powder, fine crystals, lyophilizates, etc. and dissolved in water immediately before use. Stabilizer may be added. Moreover, the compounds (I) can be administered orally together with pharmaceutical components such as diluent (e.g., starch, sucrose, lactose, calcium carbonate, kaolin), lubricant (e.g., stearic acid, sodium benzoate, boric acid, silica, polyethylene glycol) in a form of powder, tablets, granules, capsules, troches and dry syrup.

The compounds (I) are generally administered orally at 500mg-10g dosage per adult 1-3 times a day in treatment of tumors. The dosage, however, may preferably be increased or decreased optionally according to the age,

state, clinical history, etc. of the patient.

The following examples are provided to further illustrate this invention.

Example 1

A) dl-5α-Bromo-5β-fluoro-1,2,3,4,5,6-hexahydro-2,4-dioxo-6β-(2-propenyloxy)pyrimidine (2) :

i)

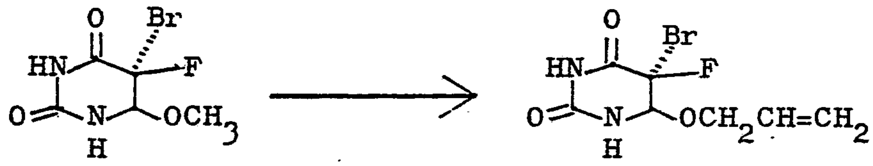

(1) (2)

dl-5α-Bromo-5β-fluoro-1,2,3,4,5,6-hexahydro-6β-methoxy-2,4-dioxopyrimidine (1)* (89.1g, 0.37mol), 2-propen-1-ol (450ml) and methanesulfonic acid (0.2ml) are placed in a flask equipped with a Dien-Stark water separator charged with Molecular sieves 4A, and the mixture is stirred under reflux with heating for about 6 hours. The crystalline residue after removal of an excess amount of 2-propen-1-ol under reduced pressure is recrystallized from acetone-ether to give the titled compound (2) (80.7g). Yield : 81.7%, m.p. 196-197°C.

ii)

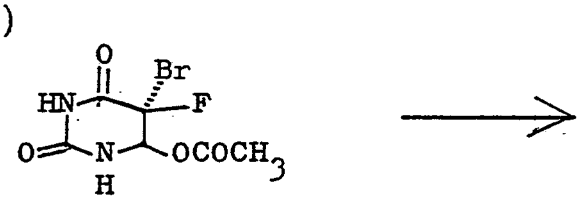

(2)

(3)

A mixture consisting of dl-6β-acetoxy-5α-bromo-5β-fluoro-1,2,3,4,5,6-hexahydro-2,4-dioxopyrimidine (3)* (2.69g, 10 mmol), 2-propen-1-ol (30ml) and methanesulfonic acid (0.1 ml) is stirred under reflux with heating for about 2 hours. After termination of the reaction, the mixture is cooled with ice, and sodium hydrogencarbonate (400mg) is added thereto followed by stirring for 30 minutes. After removal of the insoluble material by filtration, an excess amount of 2-propen-1-ol is distilled off under reduced pressure to give crystalline residue, which is recrystallized from aceton-ether to give the titled compound (2) (1.73g). Yield : 65%. m.p. 196-197°C.

*) Note

dl-5α-Bromo-5β-fluoro-1,2,3,4,5,6-hexahydro-6β-methoxy-2,4-dioxopyrimidine and dl-6β-acetoxy-5α-bromo-5β-fluoro-1,2,3,4,5,6-hexahydro-2,4-dioxopyrimidine were produced according to the methods described in the following literature.

R.Duschinsky, T.Gabriel, W.Tautz, A. Nussbaum, M.Hoffer, E.Grunberg, J.H.Burchenal and J.J.Fox, J.Med.Chem., 10, 47 (1967)

B) dl-5α-Bromo-5β-fluoro-6β-(2-hydroxy-2-methoxyethoxy)-1,2,3,4,5,6-hexahydro-2,4-dioxopyrimidine (4) :

(2) 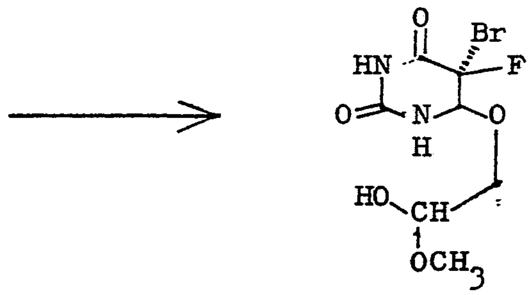 (4)

dl-5β-Fluoro-1,2,3,4,5,6-hexahydro-6β-(2-propenyloxy)-2,4-dioxopyrimidine (2) (10g, 37mmol) is dissolved in a mixture consisting of dichloromethane (200ml) and methanol (100ml), cooled to -78°C in a dry ice-acetone bath, and then the mixture is ozonized by introducing ozone gas. After the reaction mixture turns blue, dimethylsulfide (13.7ml) is added thereto, and the mixture is warmed up to 0°C and allowed to react for about 1 hour. The crystalline residue after removal of the solvent by distillation is washed with dichloromethane to give the titled compound (4) (9.7g). Yield : 86.4%. m.p. 110-116°C.

C) dl-8α-Bromo-8β-fluoro-3α-hydroxy-2,3,6,7,8,8aα-hexahydro-5,7-dioxo-5H-oxazolo[3,2-c]pyrimidine (5) :

(4) 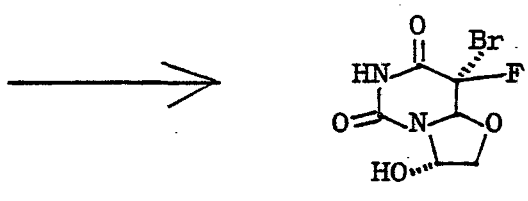 (5)

Molecular sieves 4A (100g) is added to a solution of dl-5α-bromo-5β-fluoro-6β-(2-hydroxy-2-methoxyethoxy)-1,2,3,4,5,6-hexahydro-2,4-dioxopyrimidine (4) (24g, 80mmol) in tetrahydrofuran (530ml), and the mixture is

stirred at room temperature for 4 hours. Molecular sieves 4A is filtrated off, and the solvent is distilled off. The resulting residue is crystallized from a mixture of benzene-ethyl acetate (2:1) to give the titled compound (5) (16.1g). Yield : 75.3%. m.p. 142-143°C.

Example 2

A) dl-5β-Fluoro-1,2,3,4,5,6-hexahydro-6β-(2-propenyloxy)-2,4-dioxopyrimidine (6) :

(2)

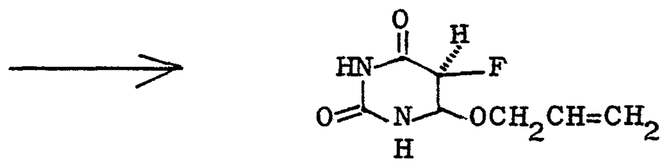

(6)

A solution of 86% potassium hydroxide (1.06g, 22mmol) dissolved in 2-propen-1-ol (25ml) is cooled with ice, and hydrogen sulfide (about 700mg, 20.6 mmol) is absorbed therein. dl-5α-Bromo-5β-fluoro-1,2,3,4,5,6-hexahydro-6β-(2-propenyloxy)-2,4-dioxopyrimidine (2) (4.33g, 16.2mmol) is slowly added thereto and allowed to stand at 0°C for 10 minutes, at room temperature for 30 minutes, and at 65°C for 20 minutes successively. The reaction mixture is cooled to room temperature, and the insoluble material is filtrated off. Evaporation of the filtrate under reduced pressure gives crystalline residue, which is washed with iced water to give the titled compound (6) (2.0g). Yield : 66%. m.p. 171-172°C.

B) dl-5β-Fluoro-6β-(2-hydroxy-2-methoxyethoxy)-1,2,3,4,5,6-hexahydro-2,4-dioxopyrimidine (7) :

(6)

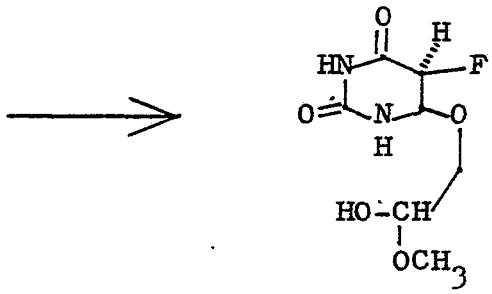

(7)

dl-5β-Fluoro-1,2,3,4,5,6-hexahydro-6β-(2-propenyloxy)-2,4-dioxopyrimidine (6) (3.85g, 20.4mmol) is dissolved in a mixture consisting of dichloromethane (150ml) and methanol (150ml), cooled to -78°C in a dry ice-acetone bath, and then ozonized by introducing ozone gas. When the reaction mixture turns blue, supply of ozone is stopped, and an excess of ozone is exhousted by introducing nitrogen gas. Dimethylsulfide (40 ml) is added thereto, and the reaction mixture is warmed up to 0°C and allowed to stand overnight. The resulting crystals are collected by filtration to give the titled compound (7) (2.24g). Yield : 49.2%. m.p. 176-178°C. The mother liquor is evaporated, and the residue is purified by chromotography over silica gel employing a mixture of benzene-ethyl acetate (1:2) as eluent to give dl-8β-fluoro-3α-hydroxy-2,3,6,7,8,8aα-hexahydro-5,7-dioxo-5H-oxazolo[3,2-c]pyrimidine (8) (359mg). Yield : 9.2%.

C) dl-8β-Fluoro-3α-hydroxy-2,3,6,7,8,8aα-hexahydro-5,7-dioxo-5H-oxazolo[3,2-c]pyrimidine (8) :

(7) 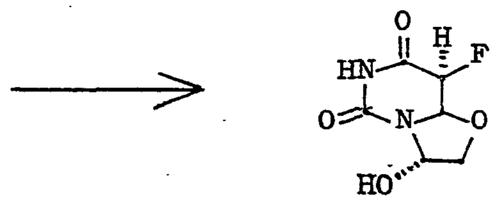 (8)

Molecular sieves 5A (about 2g) is added to a solution of dl-5β-fluoro-6β-(2-hydroxy-2-methoxyethoxy)-1,2,3,4,5,6-hexahydro-2,4-dioxopyrimidine (7) (520mg, 2.34 mmol) in tetrahydrofuran (30ml) and stirred at room temperature for 3 hours. Removal of Molecular sieves 5A by filtration and evaporation of the solvent give crystalline residue, which is recrystallized from ethyl acetate-ether to give the titled compound (8) (405mg). Yield : 91%. m.p. 188-191°C.

Alternatively, the titled compound (8) is obtained in high yield from dl-5β-fluoro-6β-(2-hydroxy-2-methoxyethoxy)-1,2,3,4,5,6-hexahydro-2,4-dioxopyrimidine on elimination of methanol by chromatography over silica gel employing a mixture of benzene-ethyl acetate (1:2) as eluent, as mentioned in Example 2-B).

Example 3

dl-3α-Acetoxy-8β-fluoro-2,3,6,7,8,8aα-hexahydro-5,7-dioxo-5H-oxazolo[3,2-c]pyrimidine (9) :

(8) 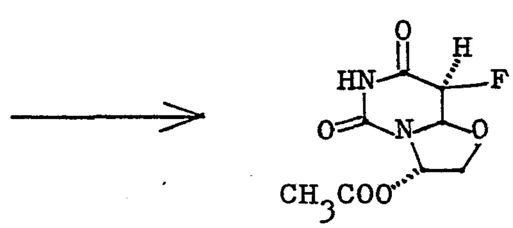 (9)

Acetic anhydride (3ml) and pyridine (0.5ml) are added to a solution of dl-8β-fluoro-3α-hydroxy-2,3,6,7,8,8aα-hexahydro-5,7-dioxo-5H-oxazolo[3,2-c]pyrimidine (8) (730mg, 3.8mmol) in tetrahydrofuran (5ml), and the mixture is allowed to react at room temperature for 15 hours. The crystalline residue obtained after removal of an excess of reagents and the solvent is recrystallized from acetone-ether to give the titled compound (9) (700mg).

Yield : 78.5%. m.p. 173-174°C.

<u>Example 4</u>

dl-8β-Fluoro-2,3,6,7,8,8aα-hexahydro-3α-octanoyloxy-5,7-dioxo-5H-oxazolo[3,2-c]pyrimidine (10) :

(8)

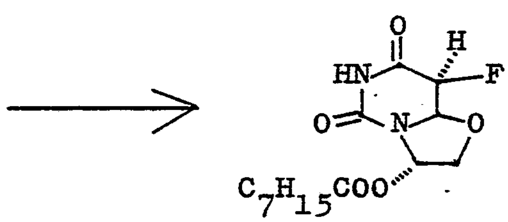

(10)

Octanoic anhydride (11.5g, 43mmol) and pyridine (3.48ml) are added to a solution of dl-8β-fluoro-3α-hydroxy-2,3,6,7,8,8aα-hexahydro-5,7-dioxo-5H-oxazolo[3,2-c]-pyrimidine (8) (2.36g, 12.4mmol) in tetrahydrofuran (50 ml), and the mixture is allowed to react at room temperature for 4 hours. After removal of an excess amount of reagents and the solvent, the product is purified by chromatography over silica gel employing a mixture of benzene-ethyl acetate (4:1) as eluent to give the titled compound (10) (2.61g).

Yield : 67%. m.p. 58-61°C.

Example 5

dl-8β-Fluoro-2,3,6,7,8,8aα-hexahydro-5,7-dioxo-3α-trimethylacetoxy-5H-oxazolo[3,2-c]pyrimidine (11) :

(8)

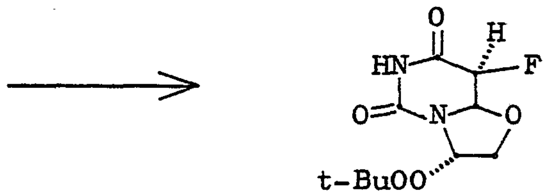

(11)

dl-8β-Fluoro-3α-hydroxy-2,3,6,7,8,8aα-hexahydro-5,7-dioxo-5H-oxazolo[3,2-c]pyrimidine (8) (1.9g, 10mmol) is dissolved in a mixture solution (70ml) consisting of acetonitrile and tetrahydrofuran (1:1), and pivalic anhydride (16.2ml, 80mmol) is added, and then cooled to 0°C. After tin tetrachloride (1.17ml, 10mmol) is added at 0°C in dropwise fashion, the reaction mixture is warmed up to room temperature and stirred for 2 hours. Anhydrous sodium hydrogencarbonate (4.2g, 50mmol) and a small amount of water are added thereto, and the mixture is allowed to react at room temperature for 30 minutes. The insoluble material is filtrated off, and the solvent is distilled off. The product is purified by chromatography on a silica gel column employing a mixture of benzene-ethyl acetate (4:1) as eluent to give the titled compound (11) (270mg).

Yield : 10%. m.p. 174-175°C.

Example 6

dl-3α-Acetoxy-8α-bromo-8β-fluoro-2,3,6,7,8,8aα-hexahydro-5,7-dioxo-5H-oxazolo[3,2-c]pyrimidine (12) :

(5) 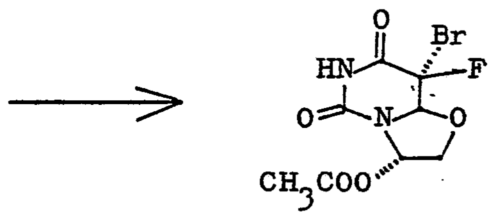 (12)

dl-8α-Bromo-8β-fluoro-3α-hydroxy-2,3,6,7,8,8aα-hexahydro-5,7-dioxo-5H-oxazolo[3,2-c]pyrimidine (5) (2.8g, 10.4mmol) is dissolved in acetic anhydride (200ml), pyridine (10ml) is added thereto, and then the mixture is allowed to stand at room temperature overnight. After removal of an excess amount of the reagent under reduced pressure, the product is purified by chromatography on a silica gel column employing a mixture of benzene-ethyl acetate (1:1) as eluent to give the titled compound (12) (2.09g). Yield : 65%. m.p. 128-131°C (Crystallized from benzene).

<u>Example 7</u>

dl-8α-Bromo-8β-fluoro-2,3,6,7,8,8aα-hexahydro-3α-octanoyloxy-5,7-dioxo-5H-oxazolo[3,2-c]pyrimidine (13) :

(5) 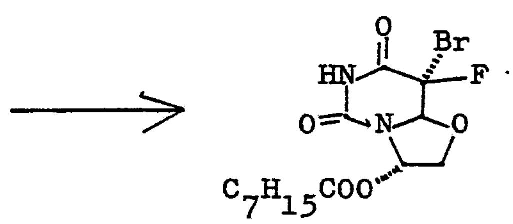 (13)

Octanoic anhydride (11.6g, 42.8mmol) and pyridine (1.73ml, 21.4mmol) are added to a solution of dl-8α-bromo-

8β-fluoro-3α-hydroxy-2,3,6,7,8,8aα-hexahydro-5,7-dioxo-5H-oxazolo[3,2-c]pyrimidine (5) (2.89g, 10.7mmol) in tetrahydrofuran (50ml), and the mixture is allowed to stand at room temperature overnight. The product obtained after removal of an excess amount of reagents and the solvent by distillation is purified by chromatography over silica gel employing a mixture of benzene-ethyl acetate (4:1) as eluent to give the titled compound (13) (2.94g). Yield : 69.5%. Oily substance.

Example 8

dl-8α-Bromo-8β-fluoro-2,3,6,7,8,8aα-hexahydro-5,7-dioxo-3α-trimethylacetoxy-5H-oxazolo[3,2-c]pyrimidine (14) :

(5)

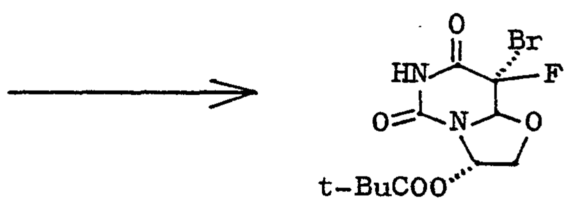

(14)

A) dl-8α-Bromo-8β-fluoro-3α-hydroxy-2,3,6,7,8,8aα-hexahydro-5,7-dioxo-5H-oxazolo[3,2-c]pyrimidine (5) (5.26g, 19.6mmol) and pivalic anhydride (31.8ml, 157mmol) are dissolved in a mixture of tetrahydrofuran (50ml) and acetonitrile (50ml), and then tin tetrachloride (2.29ml, 19.6 mmol) is added under ice cooling in dropwise fashion. The mixture is allowed to stand at room temperature for additional 2 hours, and then sodium hydrogencarbonate (8.23g, 98mmol) and a small amount of water are added and stirred

well at room temperature for 30 minutes. The insoluble material is filtrated off, and the filtrate is evaporated to dryness under reduced pressure. The oily residue is purified by chromatography over silica gel employing a mixture of benzene-ethyl acetate (4:1) as eluent to give the titled compound (14) (2.79g). Yield : 40.3%.

m.p. 188-189°C (Recrystallized from ether-petroleum ether).

B) dl-8α-Bromo-8β-fluoro-3α-hydroxy-2,3,6,7,8,8aα-hexahydro-5,7-dioxo-5H-oxazolo[3,2-c]pyrimidine (5) (5.25g, 19.5mmol), pivalic anhydride (34.2g, 183mmol) and pyridine (1.89ml, 23.4mmol) are dissolved in tetrahydrofuran (150 ml), and dimethylaminopyridine (1.19g, 9.8mmol) is added, and the mixture is allowed to stand at 65°C for 1.5 hours. After removal of an excess amount of reagents and the solvent under reduced pressure, the product is extracted with a mixture of ethyl acetate-acetonitrile. The organic layer is washed with an aqueous sodium hydrogencarbonate and saturated brine, dried on magnesium sulfate, and evaporated. The product is purified by chromatography over silica gel employing a mixture of benzene-ethyl acetate (4:1) as eluent to give the titled compound (14) (2.45g). Yield : 36%.

Example 9

dl-3α-(tert-Butyldimethylsilyloxy)-8α-bromo-8β-fluoro-2,3,6,7,8,8aα-hexahydro-5,7-dioxo-5H-oxazolo[3,2-c]-

pyrimidine (15) :

(5) 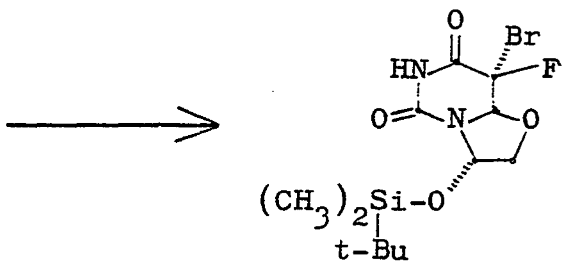 (15)

To a solution of tert-butyldimethylsilylimidazolide prepared by adding imidazole (2.39g, 35.1mmol) to a solution of tert-butyldimethylsilyl chloride (5.29g, 35.1 mmol) in dimethylformamide (30ml) is added a solution of dl-8α-bromo-8β-fluoro-3α-hydroxy-2,3,6,7,8,8aα-hexahydro-5,7-dioxo-5H-oxazolo[3,2-c]pyrimidine (5) (8.57g, 31.9mmol) in dimethylformamide (10ml), and the mixture is allowed to stand at room temperature for 17 hours. The product is extracted with ethyl acetate. The ethyl acetate layer is washed with saturated brine, dried on magnesium sulfate, and evaporated. The product is purified by chromatography over silica gel employing a mixtrue of ethyl acetate-benzene (1:2) as eluent to give the titled compound (15) (4.77g). Yield : 39%. m.p. 152-153°C.

<u>Example 10</u>

Catalytic hydrogenation of dl-3α-(tert-butyldimethylsilyloxy)-8α-bromo-8β-fluoro-2,3,6,7,8,8aα-hexahydro-5,7-dioxo-5H-oxazolo[3,2-c]pyrimidine (15) —— dl-3α-(tert-butyldimethylsilyloxy)-8α-fluoro-2,3,6,7,8,8aα-hexahydro-5,7-dioxo-5H-oxazolo[3,2-c]pyrimidine (16), dl-3α-(tert-

butyldimethylsilyloxy)-8β-fluoro-2,3,6,7,8,8aα-hexahydro-5,7-dioxo-5H-oxazolo[3,2-c]pyrimidine (17) and dl-5-fluoro-1-(2-hydroxy-1-tert-butyldimethylsilyloxyethyl)-1,2,3,4-tetrahydro-2,4-dioxopyrimidine (18) :

(15)

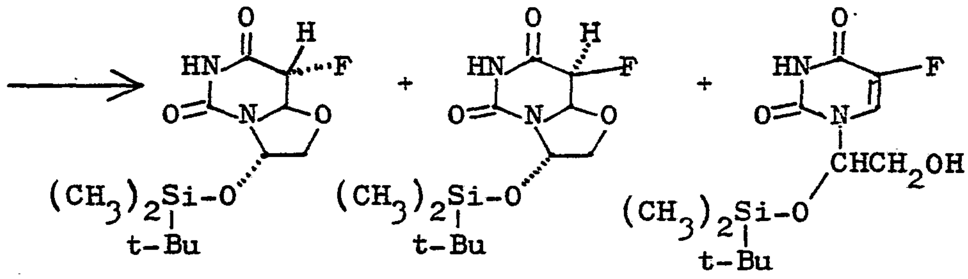

+ +

(16) (17) (18)

To a solution of dl-3α-(tert-butyldimethylsilyloxy)-8α-bromo-8β-fluoro-2,3,6,7,8,8aα-hexahydro-5,7-dioxo-5H-oxazolo[3,2-c]pyrimidine (15) (4.71g, 12.3mmol) in tetrahydrofuran (50ml) are added sodium acetate (1.11g, 13.5mmol) and 10% palladium-carbon (470mg), and the resulting suspension is hydrogenated under vigorous stirring in hydrogen atmosphere. After about 4 hours a theoretical amount (275ml) of hydrogen gas is absorbed; the insoluble material is filtrated off, and then the solvent is distilled off. The product is separated by chromatography on a silica gel column employing a mixture of benzene-ethyl acetate (4:1) as eluent to give the compound (17) (245mg) as the first fraction. Yield : 8.1%. m.p. 132-133°C (Recrystallized from methylene chloride-petroleum ether). The compound (16) (1.98g) is obtained as the main product from the next

fraction. Yield : 52.8%. m.p. 165-166°C. (Recrystallized from methylene chloride-petroleum ether). The compound (18) (874mg) is obtained from the polar fraction. Yield : 23.4%. m.p. 176-179°C. (Recrystallized from methylene chloride-petroleum ether).

Example 11

dl-8α-Fluoro-3α-hydroxy-2,3,6,7,8,8aα-hexahydro-5,7-dioxo-5H-oxazolo[3,2-c]pyrimidine (19) :

(16) 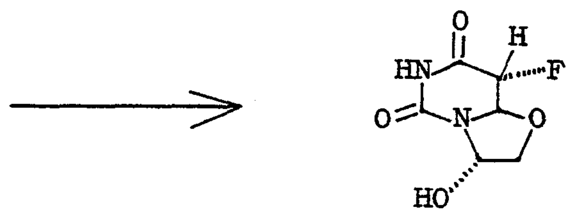 (19)

To a solution of dl-3α-(tert-butyldimethylsilyloxy)-8α-fluoro-2,3,6,7,8,8aα-hexahydro-5,7-dioxo-5H-oxazolo-[3,2-c]pyrimidine (16) (1.585g, 5.2mmol) in acetonitrile (6.6ml) is added 46% hydrofluoric acid aqueous solution (229μl), and the mixture is allowed to stand at room temperature for 2 hours. The solvent is evaporated to dryness under reduced pressure, and the product is purified by chromatography over silica gel employing a mixture of benzene-ethyl acetate (1:1) as eluent and recrystallized from acetone-ether to give the titled compound (19) (753mg). Yield : 76%. m.p. 153-158°C.

Example 12

dl-3α-Acetoxy-8α-fluoro-2,3,6,7,8,8aα-hexahydro-5,7-dioxo-5H-oxazolo[3,2-c]pyrimidine (20) and dl-5-fluoro-1-(2-hydroxy-1-acetoxyethyl)-1,2,3,4-tetrahydro-2,4-dioxopyrimidine (21) :

(12) 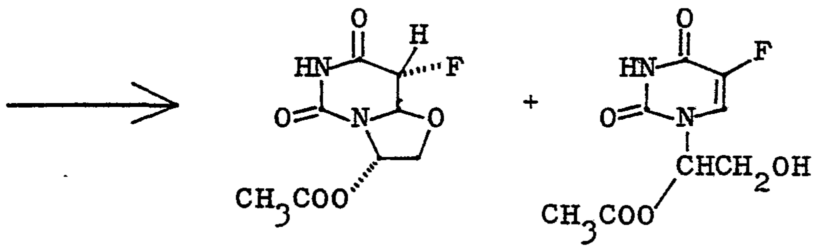 +

(20) (21)

Anhydrous sodium acetate (946mg, 11.5mmol) and 10% palladium-carbon (300mg) are suspended in a solution of dl-3α-acetoxy-8α-bromo-8β-fluoro-5,7-dioxo-2,3,6,7,8,8aα-hexahydro-5H-oxazolo[3,2-c]pyrimidine (12) (2.99g, 9.6mmol) dissolved in tetrahydrofuran (30ml), and the mixture is catalytically hydrogenated under vigorous stirring in hydrogen atmosphere. After about 3 hours a theoretical amount (215ml) of hydrogen gas is absorbed; the insoluble material is filtrated off, washed with acetonitrile thoroughly, and then the filtrate is evaporated to dryness. The resulting crystalline residue is recrystallized from acetone-methylene chloride to give the titled compound (20) (1.08g). The mother liquor is separated by chromatography on a silica gel column employing a mixture of benzene-ethyl acetate (1:1-1:2) as eluent to give the titled compound (20) (0.49g) as an additional crop (Total yield of the compound (20) : 1.57g; Yield : 69%). m.p. 182-185°C.

Additionally the compound (21) (0.17g) is obtained from the polar fraction. Yield : 7.6%. m.p. 280-285°C (Recrystallized from acetone).

Example 13

dl-8α-Fluoro-2,3,6,7,8,8aα-hexahydro-3α-octanoyloxy-5,7-dioxo-5H-oxazolo[3,2-c]pyrimidine (22) and dl-5-fluoro-1-(2-hydroxy-1-octanoyloxyethyl)-1,2,3,4-tetrahydro-2,4-dioxopyrimidine (23) :

(13) 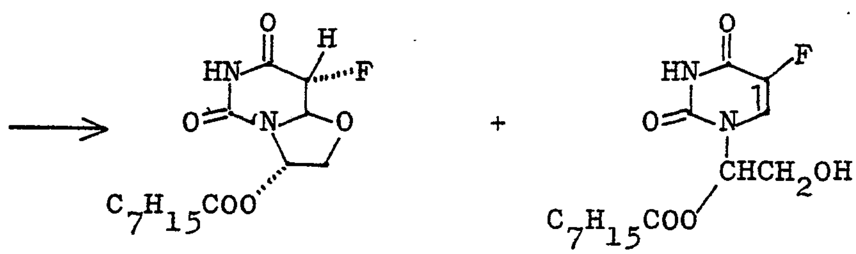

+

(22) (23)

Anhydrous sodium acetate (1.22g, 14.9mmol) and 10% palladium-carbon (400mg) are suspended in a solution of dl-8α-bromo-8β-fluoro-2,3,6,7,8,8aα-hexahydro-3α-octanoyloxy-5,7-dioxo-5H-oxazolo[3,2-c]pyrimidine (13) (2.94g, 7.4mmol) dissolved in tetrahydrofuran (70ml), and the mixture is catalytically hydrogenated under vigorous stirring in hydrogen atmosphere. After about 4 hours a theoretical amount (167ml) of hydrogen gas is absorbed; the insoluble material is filtrated off, washed with acetonitrile thoroughly, and then the filtrate is evaporated. The resulting crystalline residue is recrystallized from ether-petroleum ether to give the titled compound (22) (0.9g) as crystals

of the first crop. Moreover, the mother liquor is separated by chromatography over silica gel employing a mixture of benzene-ethyl acetate (4:1-1:1) as eluent to give the titled compound (22) (0.6g) as crystals of the second crop. Total yield of the compound (22) : 1.50g. Yield : 63.7%. m.p. 138-141°C. Additionally, the compound (23) (0.16g) is obtained from the polar fraction. Yield : 6.8%. m.p. 107-111°C (Recrystallized from acetone-ether).

Example 14

Catalytic hydrogenation of dl-8α-bromo-8β-fluoro-2,3,6,7,8,8aα-hexahydro-5,7-dioxo-3α-trimethylacetoxy-5H-oxazolo-[3,2-c]pyrimidine (14) —— dl-8α-fluoro-2,3,6,7,8,8aα-hexahydro-5,7-dioxo-3α-trimethylacetoxy-5H-oxazolo[3,2-c]-pyrimidine (24), dl-8β-fluoro-2,3,6,7,8,8aα-hexahydro-5,7-dioxo-3α-trimethylacetoxy-5H-oxazolo[3,2-c]pyrimidine (11) and dl-5-fluoro-(2-hydroxy-1-trimethylacetoxyethyl)-1,2,3,4-tetrahydro-2,4-dioxopyrimidine (25) :

(14) 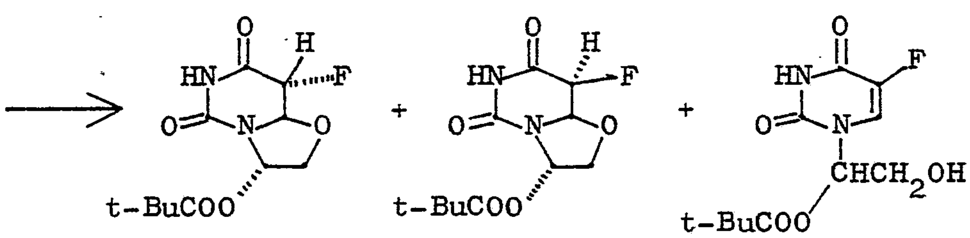

(24) (11) (25)

Anhydrous sodium acetate (1.39g, 17mmol) and 10% palladium-carbon (0.5g) are suspended to a solution of the titled compound (14) (5.0g, 14.2mmol) dissolved in tetrahydrofuran (50ml), and the mixture is catalytically hydrogenated under vigorous stirring in hydrogen atmosphere. After about 3 hours a theoretical amount (320ml) of hydrogen gas is absorbed; the insoluble material is filtrated off, and then the filtrate is evaporated. The resulting crystalline residue is recrystallized from acetone-ether to give the compound (24) (2.02g). Yield : 52.0%. The mother liquor is separated by chromatography over silica gel employing a mixture of benzene-ethyl acetate (4:1-1:1) as eluent to give the compound (11) (372mg) as the first fraction. Yield : 9.6%. m.p. 176-177°C (Recrystallized from acetone-ether). The compound (24) (480mg) is obtained as the main product from the next fraction. Total amount of the compound (24) : 2.50g. Total yield : 64.4%. m.p. 164-166°C (Recrystallized from acetone-ether). Additionally, the compound (25) (20mg) is obtained from the polar fraction. Yield : 0.5%. m.p. 151-152°C (Recrystallized from acetone-ether).

<u>Example 15</u>

A) dl-5α-Bromo-6β-(3-butenyloxy)-5β-fluoro-1,2,3,4,5,6-hexahydro-2,4-dioxopyrimidine (26) :

i)

(1)

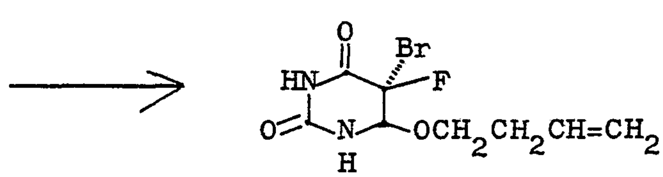

(26)

dl-5α-Bromo-5β-fluoro-1,2,3,4,5,6-hexahydro-6β-methoxy-2,4-dioxopyrimidine (1) (50g, 207mmol), 3-buten-1-ol (27ml, 310mmol) and methanesulfonic acid (5ml) are dissolved in acetonitrile (500ml), placed in a flask equipped with a Dien-Stark water separator charged with Molecular sieves 4A (60g), and then refluxed with heating for 4 hours. After cooling, the product is extracted with ether. The ether layer is washed with an aqueous sodium hydrogencarbonate and saturated brine, dried on magnesium sulfate, and the solvent is distilled off. The resulting crystals are washed with water several times and then dried to give the titled compound (26) (36g). Yield : 72%.

ii) (3) ⟶ (26)

To a solution of dl-6β-acetoxy-5α-bromo-5β-fluoro-1,2,3,4,5,6-hexahydro-2,4-dioxopyrimidine (3) (30g, 110mmol) and 3-buten-1-ol (14.6ml, 165mmol) dissolved in acetonitrile (500ml) is added methanesulfonic acid (1 ml), and the mixture is allowed to stand at 70-80°C for 3 hours. The solvent and an excess amount of the reagent are evaporated, and water is added to the residue to give crystalline product. The product is collected by filtration, washed with water several times and dried to give the titled compound (26) (21.5g). Yield : 70%. m.p. 136.5-139°C (Recrystallized from ether-petroleum ether).

B) dl-5α-Bromo-5β-fluoro-6β-(3-hydroxy-3-methoxypropoxy)-1,2,3,4,5,6-hexahydro-2,4-dioxopyrimidine (27) :

(26) 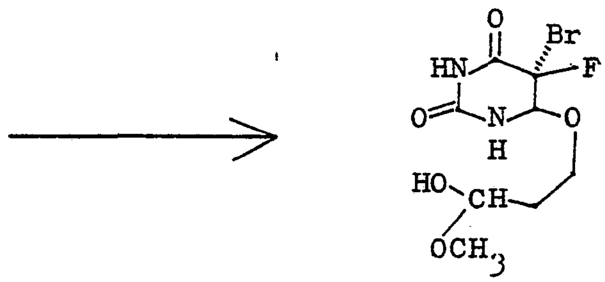 (27)

dl-5α-Bromo-6β-(3-butenyloxy)-5β-fluoro-1,2,3,4,5,6-hexahydro-2,4-dioxopyrimidine (26) (21.5g, 77mmol) is dissolved in a mixture solution of dichloromethane (400ml) and methanol (200ml), cooled to -70°C in a dry ice-acetone bath and ozonized by introducing ozone gas. When the reaction mixture turns blue, supply of ozone is stopped, and an excess amount of ozone is exhausted by introducing nitrogen gas; then dimethylsulfide (41ml) is added thereto, and the mixture is allowed to stand at 0°C for 15 hours. The product obtained by removal of the solvents is purified by chromatography on a silica gel column employing a mixture of benzene-ethyl acetate (1:1-1:2) as eluent to give the titled compound (27) (15.4g). Yield : 63%. m.p. 99-102°C.

C) dl-9α-Bromo-9β-fluoro-3,4,7,8,9,9aα-hexahydro-4α-hydroxy-6,8-dioxo-2H,6H-[1,3]-oxazino-[3,2-c]pyrimidine (28) :

(27) 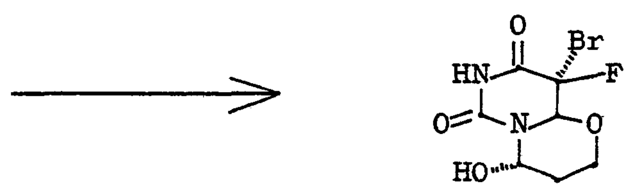 (28)

dl-5α-Bromo-5β-fluoro-6β-(3-hydroxy-3-methoxy-propoxy)-1,2,3,4,5,6-hexahydro-2,4-dioxopyrimidine (27) (5.0g, 15.9mmol) is dissolved in acetone (30ml) and water (30ml), and 60% perchloric acid aqueous solution (5ml) is added thereto; the mixture is allowed to stand at room temperature for 3 hours. After the reaction mixture is neutralized with sodium carbonate, the solvent is distilled off under reduced pressure. The remaining moisture is removed by azeotropic distillation with benzene as much as possible. Ether is added to the residue to extract the product, and sodium perchlorate which is insoluble in ether is removed by filtration. After removal of the solvent, the ether extract is purified by chromatography on a silica gel column employing a mixture of benzene-ethyl acetate (1:1) as eluent to give the titled compound (28) (2.93g). Yield : 65%. m.p. 137.5-139.5°C (Recrystallized from ether).

The mother liquor (about 9.3g) after crystallization of dl-5α-bromo-5β-fluoro-6β-(3-hydroxy-3-methoxy-propoxy)-1,2,3,4,5,6-hexahydro-2,4-dioxopyrimidine (27) obtained by the above mentioned ozonization reaction is dissolved in acetone (50ml) and water (50ml), and allowed to react with 60% aqueous perchloric acid (8.3ml) at room temperature for 4 hours. The reaction mixture is worked up in the same manner as mentioned above to give the titled compound (28)(4.19g). Overall yield of the titled compound

(28) produced from dl-5α-bromo-6β-(3-butenyloxy)-5β-fluoro-1,2,3,4,5,6-hexahydro-2,4-dioxopyrimidine (26) (21.5g) is 13.2g. Yield : 60.6%.

Example 16

A) dl-5β-Fluoro-6β-(3-butenyloxy)-1,2,3,4,5,6-hexahydro-2,4-dioxopyrimidine (29) :

(26)

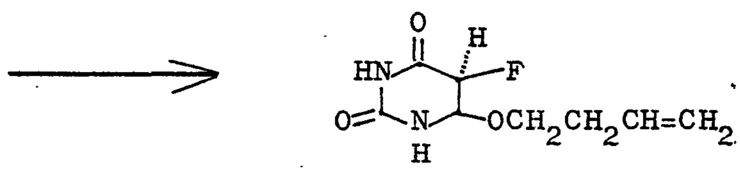

(29)

A solution of 86% potassium hydroxide (5.05g, 77.4mmol) dissolved in methanol (180ml) is cooled to -5°C, and hydrogen sulfide (3.34g, 98.3mmol) is absorbed therein. dl-5α-Bromo-6β-(3-butenyloxy)-5β-fluoro-1,2,3,4,5,6-hexahydro-2,4-dioxopyrimidine (26) (22.9g, 81.5mmol) is added thereto and allowed to react at -5°C for 10 minutes, at room temperature for 30 minutes, and then at 60°C for 10 minutes, successively. After cooling, the reaction mixture is neutralized with sodium hydrogencarbonate, and the insoluble material is filtrated off and washed with acetone. The filtrate is evaporated, and the crystalline residue is crystallized from acetone-petroleum ether to give the titled compound (29) (8.2g). Yield : 53%. m.p. 167-169°C.

B) dl-5β-Fluoro-6β-(3-hydroxy-3-methoxypropoxy)-1,2,3,4,5,6-hexahydro-2,4-dioxopyrimidine (30) :

(29) 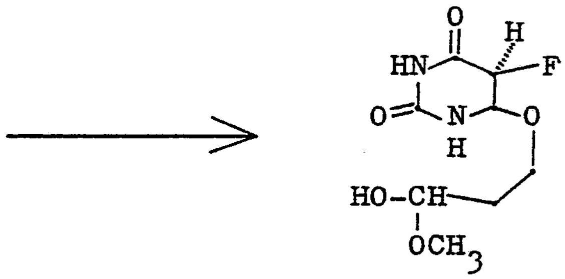 (30)

dl-5β-Fluoro-6β-(3-butenyloxy)-1,2,3,4,5,6-hexahydro-2,4-dioxopyrimidine (29) (10.2g, 50.4mmol) is dissolved in a mixture of dichloromethane (200ml) and methanol (100ml), cooled to -70°C in a dry ice-acetone bath, and ozonized by introducing ozone gas. When the reaction mixture turns blue, supply of ozone is stopped, and an excess amount of ozone is exhausted by introducing nitrogen gas. Dimethyl sulfide (32ml) is added thereto, and the mixture is allowed to stand at 0°C for 1 hour. The solvent is removed by distillation, and the residue is dissolved in a mixture of benzene-ethyl acetate (1:2), passed through a silica gel column to remove dimethylsulfoxide and then evaporated to give the titled compound (30) (7.78g) as an oily crude product. The obtained product containing a small amount of the compound (30) is subjected to the following reaction.

C) dl-9β-Fluoro-4α-hydroxy-3,4,7,8,9,9aα-hexahydro-6,8-dioxo-2H,6H-[1,3]-oxazino[3,2-c]pyrimidine (31) :

(30)

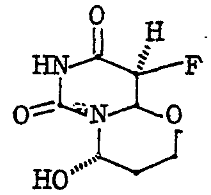

(31)

The crude dl-5β-fluoro-6β-(3-hydroxy-3-methoxypropoxy)-1,2,3,4,5,6-hexahydro-2,4-dioxopyrimidine (30) obtained in B) is dissolved in a mixture of acetone (50ml) and water (50ml), to which conc. sulfuric acid (1 ml) is added, and allowed to stand at room temperature for 1 hour. The reaction mixture is neutralized with sodium carbonate, and the solvent is distilled off under reduced pressure. The residue id extracted with acetone to remove sodium sulfate produced, and the acetone extract is passed through a short column of silica gel. The eluate with acetone is evaporated, and the residue is recrystallized from acetone-ether to give the titled compound (31)(3.73g). Yield from the compound (29) : 36%. m.p. 126-128°C.

Example 17

dl-4α-(tert-Butyldimethylsilyloxy)-7-tert-butyldimethylsilyl-9α-bromo-9β-fluoro-3,4,7,8,9,9aα-hexahydro-6,8-dioxo-2H,6H-[1,3]-oxazino[3,2-c]pyrimidine (32) :

(28)

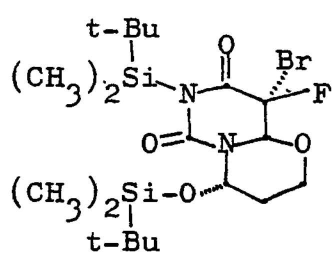

(32)

To a solution of tert-butyldimethylsilylimidazolide prepared by adding imidazole (5.61g, 82.5mmol) to a solution of tert-butyldimethylsilyl chloride (8.29g, 55 mmol) in dimethylformamide (120ml) is added dl-9α-bromo-9β-fluoro-3,4,7,8,9,9aα-hexahydro-4α-hydroxy-6,8-dioxo-2H,6H-[1,3]-oxazino[3,2-c]pyrimidine (28) (6.25g, 22mmol), and the mixture is allowed to stand at room temperature for 2.5 days. The product is extracted with ethyl acetate, and the ethyl acetate layer is washed with water, dried on magnesium sulfate, and evaporated. The residue is purified by chromatography on a silica gel column employing a mixture of benzene-ethyl acetate (9:1) as eluent to give the titled compound (32) (6.06g). Yield : 54%.
m.p. 152-155°C.

Example 18

Catalytic hydrogenation of dl-4α-(tert-butyldimethylsilyloxy)-7-tert-butyldimethylsilyl-9α-bromo-9β-fluoro-3,4,7,8,9,9aα-hexahydro-6,8-dioxo-2H,6H-[1,3]-oxazino-[3,2-c]pyrimidine (32) :

(32)

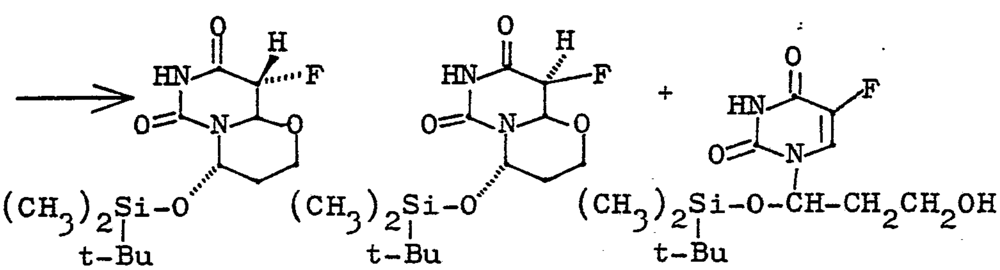

+

(33) (34) (35)

To a solution of the compound (32) (6.06g,11.9

mmol) dissolved in tetrahydrofuran (60ml) are added 10% palladium-carbon (300mg) and anhydrous sodium acetate (1.38g, 16.8mmol), and the mixture is hydrogenated under vigorous stirring in hydrogen atmosphere. After about 4 hours a theoretical amount (533ml) of hydrogen gas is absorbed; insoluble material is filtrated off and washed with acetonitrile. The filtrate is evaporated, and the residue is separated by chromatography over silica gel employing a mixture of benzene-ethyl acetate (4:1-1:2) as eluent to give a mixture (3.06g, Yield : 81%) of dl-4α-(tert-butyldimethylsilyloxy)-9α-fluoro-3,4,7,8,9,9aα-hexahydro-6,8-dioxo-2H,6H-[1,3]-oxazino[3,2-c]pyrimidine (33) and dl-4α-(tert-butyldimethylsilyloxy)-9β-fluoro-3,4,7,8,9,9aα-hexahydro-6,8-dioxo-2H,6H-[1,3]-oxazino[3,2-c]pyrimidine (34) (about 3:2), and from the polar fraction dl-5-fluoro-1-(3-hydroxy-1-tert-butyldimethylsilyloxypropyl)-1,2,3,4-tetrahydro-2,4-dioxopyrimidine (35) (331 mg, Yield : 8.7%). m.p. 131-133°C.

Example 19

dl-9β-Fluoro-3,4,7,8,9,9aα-hexahydro-4α-hydroxy-6,8-dioxo-2H,6H-[1,3]-oxazino[3,2-c]pyrimidine (31), dl-9α-fluoro-3,4,7,8,9,9aα-hexahydro-4α-hydroxy-6,8-dioxo-2H,6H-[1,3]-oxazino[3,2-c]pyrimidine (36) and dl-9α-fluoro-3,4,7,8,9,9aα-hexahydro-4β-hydroxy-6,8-dioxo-2H,6H-[1,3]-oxazino-[3,2-c]pyrimidine (37) :

(33) + (34)

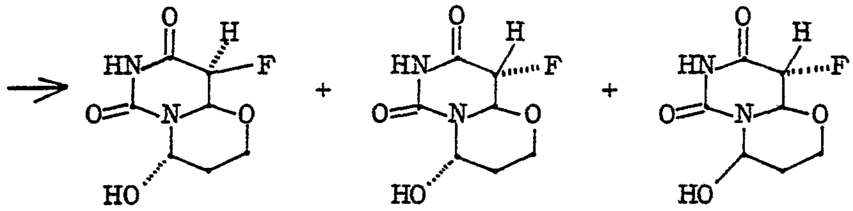

(31) (36) (37)

To a solution of a mixture (2.64g, 8.29mmol) of dl-9α and 9β-fluoro-4α-(tert-butyldimethylsilyloxy)-3,4,7,8,9,9aα-hexahydro-6,8-dioxo-2H,6H-[1,3]-oxazino[3,2-c]-pyrimidine (33) and (34) dissolved in acetonitrile (12ml) is added 4.6% aqueous hydrofluoric acid (381μl) and allowed to stand at room temperature for 2 hours. The solvent is removed by distillation to give residue, of which the acetone soluble part (1.61g) is separated by chromatography on a silica gel column employing a mixture of benzene-ethyl acetate (1:2) as eluent to give at first the titled compound (37) (m.p. 232-234°C, recrystallized from acetone-ether) and subsequently the titled compound (36) (m.p. 148-152°C, recrystallized from acetone-ether) and finally the titled compound (31) (m.p. 126-128°C, recrystallized from acetone-ether).

Example 20

dl-4α-Acetoxy-9α-bromo-9β-fluoro-3,4,7,8,9,9aα-hexahydro-6,8-dioxo-2H,6H-[1,3]-oxazino[3,2-c]pyrimidine (38) :

(28) 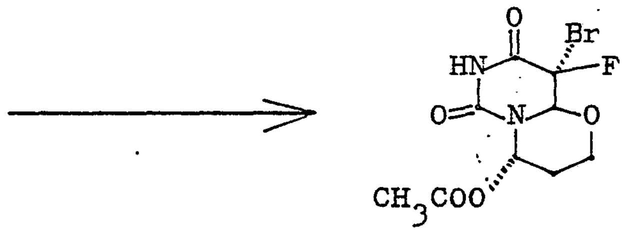 (38)

dl-9α-Bromo-9β-fluoro-3,4,7,8,9,9aα-hexahydro-4α-hydroxy-6,8-dioxo-2H,6H-[1,3]-oxazino[3,2-c]pyrimidine (28) (14.4g, 50.9mmol) is added to acetic anhydride (210 ml) and pyridine (4.1ml, 50.9mmol), and allowed to stand at room temperature for 15 hours. An excess amount of the reagents is distilled off, and the residue is purified by chromatography on a silica gel column employing a mixture of benzene-ethyl acetate (3:1) as eluent to give the titled compound (38) (10.59g). Yield : 64%. m.p. 171-174°C (Recrystallized from benzene).

Example 21

dl-4α-Acetoxy-9α-fluoro-3,4,7,8,9,9aα-hexahydro-6,8-dioxo-2H,6H-[1,3]-oxazino[3,2-c]pyrimidine (39) and dl-4α-acetoxy-9β-fluoro-3,4,7,8,9,9aα-hexahydro-6,8-dioxo-2H,6H-[1,3]-oxazino[3,2-c]pyrimidine (40) :

(38) 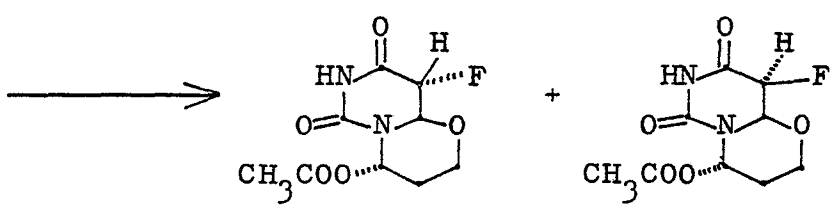

+

(39) (40)

Anhydrous sodium acetate (2.57g, 31.3mmol) and 10% palladium-carbon (500mg) are added to a solution of

dl-4α-acetoxy-9α-bromo-9β-fluoro-3,4,7,8,9,9aα-hexahydro-6,8-dioxo-2H,6H-[1,3]-oxazino[3,2-c]pyrimidine (38) (8.48g, 26.1mmol) dissolved in tetrahydrofuran (100ml), and the mixture is catalytically hydrogenated under vigorous stirring in hydrogen atmosphere. After about 4 hours a theoretical amount (585ml) of hydrogen gas is absorbed; the insoluble material is filtrated off and then washed with acetonitrile. The filtrate is evaporated, and the residue is separated by chromatography on a silica gel column employing a mixture of benzene-ethyl acetate (1:1) as eluent to give dl-4α-acetoxy-9α-fluoro-3,4,7,8,9,9aα-hexahydro-6,8-dioxo-2H,6H-[1,3]-oxazino[3,2-c]pyrimidine (39) (2.55g, Yield : 39.7%. m.p. 150-154°C) and dl-4α-acetoxy-9β-fluoro-3,4,7,8,9,9aα-hexahydro-6,8-dioxo-2H,6H-[1,3]-oxazino[3,2-c]pyrimidine (40) (1.37g, Yield.: 21.3%. m.p. 153.5-155.5°C).

Example 22

dl-9α-Bromo-9β-fluoro-3,4,7,8,9,9aα-hexahydro-4α-octanoyloxy-6,8-dioxo-2H,6H-[1,3]-oxazino[3,2-c]pyrimidine (41):

(28) 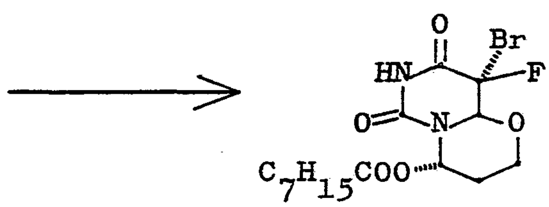 (41)

Dimethylaminopyridine (499mg, 4mmol) is added to a solution of dl-9α-bromo-9β-fluoro-3,4,7,8,9,9aα-hexahydro-4α-hydroxy-6,8-dioxo-2H,6H-[1,3]-oxazino[3,2-c]pyrimidine

(28) (2.31g, 8.1mmol), octanoic anhydride (8.83g, 32.4mmol) and pyridine (0.66ml, 8.1mmol) dissolved in tetrahydrofuran (40ml), and the mixture is allowed to stand at room temperature for 15 hours. The solvent is evaporated, and the residue is separated and purified by chromatography on a silica gel column employing a mixture of benzene-ethyl acetate (4:1) as eluent, and recrystallized from ether-petroleum ether to give the titled compound (41)(1.29g). Yield : 38.9%. m.p. 108-110°C.

Example 23

dl-9α-Bromo-9β-fluoro-3,4,7,8,9,9aα-hexahydro-6,8-dioxo-4α-trimethylacetoxy-2H,6H-[1,3]-oxazino[3,2-c]pyrimidine (42) :

(28) 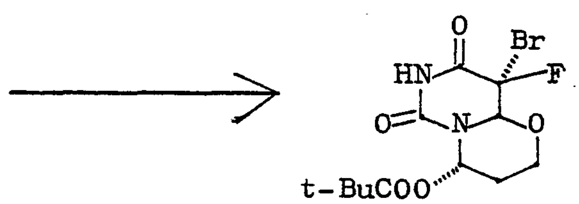 (42)

i) dl-9α-Bromo-9β-fluoro-3,4,7,8,9,9aα-hexahydro-4α-hydroxy-6,8-dioxo-2H,6H-[1,3]-oxazino[3,2-c]pyrimidine (28) (820mg, 2.9mmol) and pivalic anhydride (4.7ml, 23.2mmol) are dissolved in a mixture of tetrahydrofuran (2ml) and acetonitrile (8ml), and tin tetrachloride (0.33ml, 2.9mmol) is added in dropwise fashion under ice cooling.
The mixture is stirred at room temperature for additional 2 hours, and sodium hydrogencarbonate (1.34g, 16mmol)

and a small amount of water are added and stirred well at room temperature for 30 minutes. The insoluble material is filtrated off, and the filtrate is evaporated to dryness under reduced pressure. The oily residue is purified by chromatography over silica gel employing a mixture of benzene-ethyl acetate (2:1) as eluent to give the titled compound (42) (186mg). Yield : 16%. m.p. 178-179.5°C (Recrystallized from tetrahydrofuran-acetone).

ii) dl-9α-Bromo-9β-fluoro-3,4,7,8,9,9aα-hexahydro-4α-hydroxy-6,8-dioxo-2H,6H-[1,3]-oxazino[3,2-c]pyrimidine (28) (10g, 35.3mmol), pivalic anhydride (46ml, 226mmol) and pyridine (2.86ml, 35.3mmol) are dissolved in tetrahydrofuran (70ml), and dimethylaminopyridine (2.16g, 17.7mmol) is added thereto and allowed to stand at room temperature for 4 hours. The precipitated insoluble material is filtrated off, and the filtrate is concentrated. The product is crystallized from tetrahydrofuran-acetone to give the titled compound (42) (4.84g). Moreover, the mother liquor is separated and purified by chromatography on a silica gel column employing a mixture of benzene-acetone (2:1) to give the titled compound (42) (0.95g) as an additional crop. Total amount : 5.79g. Yield : 44.7%. m.p. 178-179.5°C (Recrystallized from tetrahydrofuran-acetone).

Example 24

dl-9α-Fluoro-3,4,7,8,9,9aα-hexahydro-6,8-dioxo-4α-tri-

methylacetoxy-2H,6H-[1,3]-oxazino[3,2-c]pyrimidine (43):

(42) 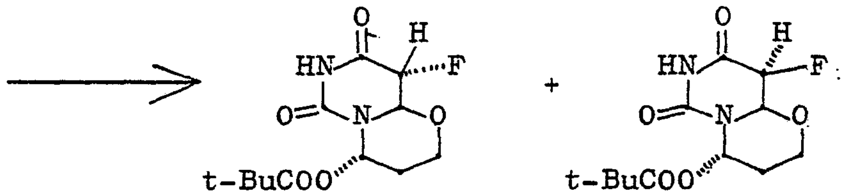

+

(43) (46)

To a solution of dl-9α-bromo-9β-fluoro-3,4,7,8,9,9aα-hexahydro-6,8-dioxo-4α-trimethylacetoxy-2H,6H-[1,3]-oxazino[3,2-c]pyrimidine (42) (6.0g, 16.3mmol) in a mixture of methanol (50ml) and tetrahydrofuran (240ml) are added anhydrous sodium acetate (2g, 24.5mmol) and 10% palladium-carbon (600mg), and the mixture is catalytically hydrogenated under vigorous stirring in hydrogen atmosphere. After about 1 hour a theoretical amount (365ml) of hydrogen gas is absorbed; the insoluble material is filtrated off and washed with acetonitrile. The solvent is distilled off, and the residue is extracted with a mixture of ethyl acetate-acetonitrile. The organic layer is washed with an aqueous sodium hydrogencarbonate solution and saturated brine and dried on magnesium sulfate, and then evaporated to dryness. The residue is recrystallized from acetone-ether to give the titled compound (43) (1.84g). Yield : 39.2%. m.p. 163-166°C. The mother liquor contains the titled compound (43) and its 9β-fluoro-epimer i.e., dl-9β-fluoro-3,4,7,8,9,9aα-hexahydro-6,8-dioxo-4α-trimethylacetoxy-2H,6H-[1,3]-oxazino[3,2-c]pyrimidine (46). The ratio of two isomers,

9α-fluoro to 9β-fluoro derivatives produced by catalytic hydrogenation is about 3:2, which is determined by NMR spectrum.

Example 25

dl-9α-Fluoro-3,4,7,8,9,9aα-hexahydro-4α-octanoyloxy-6, 8-dioxo-2H,6H-[1,3]-oxazino[3,2-c]pyrimidine (44) :

(36) 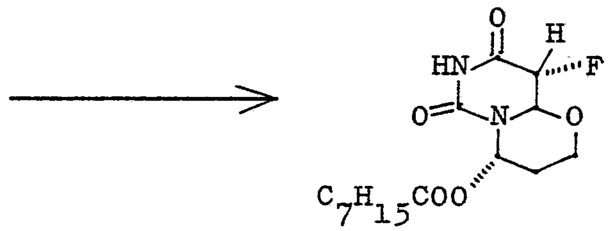 (44)

dl-9α-Fluoro-3,4,7,8,9,9aα-hexahydro-4α-hydroxy-6,8-dioxo-2H,6H-[1,3]-oxazino[3,2-c]pyrimidine (36) (408 mg, 2mmol), octanoic anhydride (2.7g, 10mmol) and pyridine (1 ml, 12.7mmol) are dissolved in tetrahydrofuran (5 ml) and allowed to stand at room temperature for 15 hours. An excess amount of reagents and the solvent are removed by distillation under reduced pressure, and the product is purified by chromatography on a silica gel column employing a mixture of benzene-ethyl acetate (4:1) as eluent to give the titled compound (44)(423mg). Yield :64%. m.p.85-86°C.

Example 26

dl-9β-Fluoro-3,4,7,8,9,9aα-hexahydro-4α-octanoyloxy-6, 8-dioxo-2H,6H-[1,3]-oxazino[3,2-c]pyrimidine (45) :

(31) 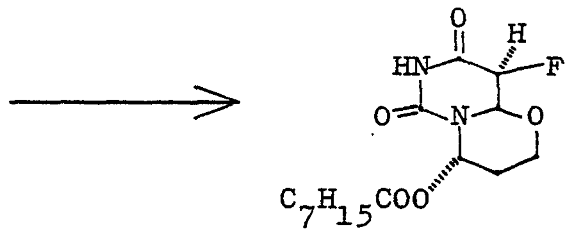 (45)

dl-9β-Fluoro-3,4,7,8,9,9aα-hexahydro-4α-hydroxy-6,8-dioxo-2H,6H-[1,3]-oxazino[3,2-c]pyrimidine (31) (100mg, 0.49 mmol), octanoic anhydride (460mg, 1.7mmol) and pyridine (0.048ml, 0.59mmol) are dissolved in tetrahydrofuran (2 ml) and allowed to stand at room temperature for 15 hours. An excess amount of reagents and the solvent are removed by distillation under reduced pressure, and the product is purified by chromatography on a silica gel column employing a mixture of benzene-ethyl acetate (4:1) as eluent to give the titled compound (45) (100mg). Yield : 62%.

Oily substance.

| Compound No. | Structural Formula | m.p., Molecular Formula | UV, IR Spectrum | NMR Spectrum ($d_6$-Acetone) |
|---|---|---|---|---|
| 2 | 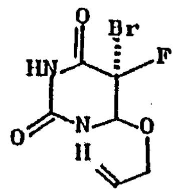 | mp 196-197°C<br>$C_7H_8BrFN_2O_3$ (267.07)<br>Anal. C H N F<br>cal. 31.48 3.02 10.49 7.11<br>F 31.21 2.96 10.47 7.57 | IR(KBr) 3250, 1754, 1717 $cm^{-1}$ | 4.27(2H,m), 5.5-5.1(3H,m), 6.3-5.6(1H,m), 8.08(1H,br), 9.87(1H,br) |
| 4 | 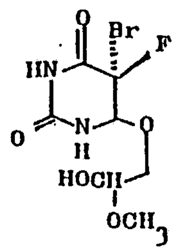 | mp 110-116°C<br>$C_7H_{10}BrFN_2O_5$ (301.086)<br>Anal. C H N F<br>cal. 27.92 3.35 9.31 6.31<br>F 28.02 3.47 9.16 6.58 | IR(KBr) 3360, 3240, 1740, 1717 $cm^{-1}$ | 3.35(3H,S), 3.68(2H,d,J=5Hz), 4.68(1H,m), 5.35(1H,d,J=4.5Hz), 8.13(1H,br), 9.83(1H,br)<br>(+$CO_3OD$) 3.35(3H,S), 3.67(2H,d,J=5Hz), 4.67(1H,t,J=5Hz),5.35(1H,S) |
| 5 | 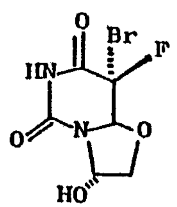 | mp 142-143°C<br>$C_6H_6BrFN_2O_4$ (269.044)<br>Anal. C H N F<br>cal. 26.78 2.25 10.41 7.06<br>F 27.08 2.42 10.25 7.16 | IR(KBr) 3450, 3225, 1740, 1720 $cm^{-1}$ | 4.03(1H,dd,J=1&9Hz), 4.37(1H,dd,J=4&9Hz), 5.78(1H,d,J=18Hz), 5.97(1H,m) |
| 6 | 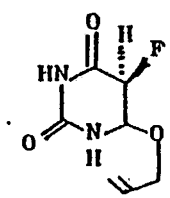 | mp 171-172°C<br>$C_7H_9FN_2O_3$ (188.162)<br>Anal. C H N F<br>cal. 44.68 4.82 14.89 10.10<br>F 44.55 4.64 14.87 10.10 | IR(KBr) 3230, 1758, 1716 $cm^{-1}$ | 4.17(2H,m), 5.5-4.9(4H,m), 6.2-5.6(1H,m), 7.9(1H,br), 9.4(1H,br) |

| Compd. No. | Structural Formula | m.p., Molecular Formula | UV, IR Spectrum | NMR Spectrum ($d_6$-Acetone) |
|---|---|---|---|---|
| 7 | 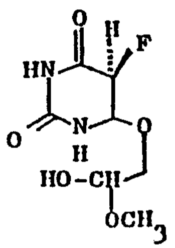 | mp 176-178°C<br>$C_7H_{11}FN_2O_5$ (222.18)<br>Anal. C H N F<br>cal. 37.84 4.99 12.61 8.55<br>F 37.58 4.76 12.83 8.69 | IR(KBr) 3330, 1740, 1725 $cm^{-1}$ | 3.35(3H,S), 3.60(2H,d,J=5Hz), 4.67(1H,m), 4.93-5.33(2H,m), 5.47(1H,dd,J=4&36Hz), 7.8(1H, br), 11.0(1H,br)<br>($+D_2O$) 3.37(3H,S), 3.61(2H,d, J=5Hz), 4.7(1H,t,J=5Hz), 5.10 (1H,dd,J=4&6Hz), 5.50(1H,dd,J= 4&40Hz) |
| 8 | 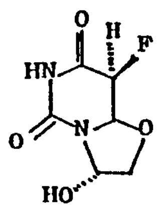 | mp 188-191°C<br>$C_6H_7FN_2O_4$ (190.136)<br>Anal. C H N F<br>cal. 37.90 3.71 14.74 9.99<br>F 37.84 3.73 14.77 9.98 | IR(KBr) 3395, 3246, 1738, 1677 $cm^{-1}$ | 3.93(1H,dd,J=2&9Hz), 4.28(1H, dd,J=4&9Hz), 4.98(1H,dd,J= 2&51Hz), 5.65(1H,dd,J=2&24Hz), 5.87(1H,m), 8.8-12(1H,br) |
| 9 | 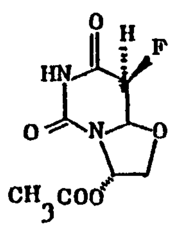 | mp 173-174°C<br>$C_8H_9FN_2O_5$(232.172)<br>Anal. C H N F<br>cal. 41.38 3.91 12.07 8.18<br>F 41.56 3.97 11.86 8.38 | IR(KBr) 3220, 1740, 1702 $cm^{-1}$ | 2.07(3H,S), 4.10(1H,dd,J=2&10 Hz), 4.40(1H,ddd,J=0.5,4&10Hz), 5.06(1H,dd,J=2&50Hz), 5.79(1H, dd,J=2&24Hz), 6.68(1H,dd,J= 2&4Hz), 10.0(1H,br) |
| 10 | 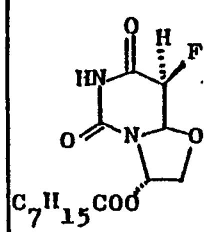 | mp 58-61°C<br>$C_{14}H_{21}FN_2O_5$ (316.33)<br>Anal. C H N F<br>cal. 53.15 6.69 8.86 6.01<br>F 53.21 6.70 8.86 6.04 | IR(KBr) 3270, 1740, 1723 $cm^{-1}$ | 0.87(3H,t,J=6Hz), 1.0-2.0(10H, m), 2.37(2H,t,J=6Hz), 4.12(1H, dd,J=2&10Hz), 4.42(1H,dd,J=4& 10Hz), 5.08(1H,dd,J=2&50Hz), 5.80(1H,dd,J=2&24Hz), 6.72(1H, dd,J=2&4Hz), 9.83(1H,br) |

| Compd. No. | Structural Formula | m.p., Molecular Formula | UV, IR Spectrum | NMR Spectrum ($d_6$-Acetone) |
|---|---|---|---|---|
| 11 | 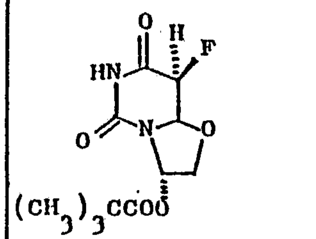 | mp 176-177°C<br>$C_{11}H_{15}FN_2O_5$ (274.25)<br>Anal. C H N F<br>cal. 48.17 5.51 10.22 6.93<br>F 48.06 5.39 10.26 7.06 | IR(KBr) 3290, 1723(br) | 1.20(9H,S), 4.08(1H,dd,J=2&10 Hz), 4.45(1H,dd,J=4&10Hz), 5.08(1H,dd,J=2&50Hz), 5.82(1H, dd,J=2&24Hz), 6.70(1H,dd,J=2& 4Hz) |
| 12 | 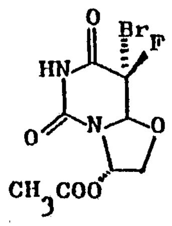 | mp 128-131°C<br>$C_8H_8BrFN_2O_5$ (311.08)<br>Anal. C H N F<br>cal. 30.89 2.59 9.00 6.11<br>F 31.24 2.67 8.92 6.50 | IR($CHCl_3$) 3370, 1745 $cm^{-1}$ | 2.10(3H,S), 4.20(1H,dd,J=1&10 Hz), 4.47(1H,ddd,J=0.5,4&10Hz), 5.75(1H,d,J=18Hz), 6.75(1H,dd, J=1&4Hz), 9.5-12.8(1H,br) |
| 13 | 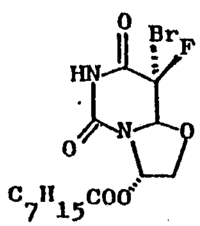 | oil<br>$C_{14}H_{20}BrFN_2O_5$ (395.236) | IR($CHCl_3$) 3370, 1740 $cm^{-1}$ | 0.90(3H,t,J=5Hz), 2.30(2H,t,J= 6Hz), 1.0-2.0(10H,m), 4.18(1H, dd,J=1&10Hz), 4.47(1H,ddd,J=0.5, 4&10Hz), 5.88(1H,d,J=19Hz), 6.77 (1H,dd,J=1&4Hz) |
| 14 | 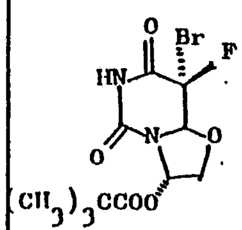 | mp 188-189°C<br>$C_{11}H_{14}BrFN_2O_5$ (353.156)<br>Anal. C H N F<br>cal. 37.41 4.00 7.93 5.38<br>F 37.35 3.81 7.87 5.58 | IR($CHCl_3$) 3370, 1740 $cm^{-1}$ | 1.20(9H,S), 4.15(1H,dd,J=1&9 Hz), 4.47(1H,ddd,J=0.5,4&9Hz), 5.95(1H,d,J=18Hz), 6.77(1H,dd, J=1&4Hz) |

| Compd. No. | Structural Formula | m.p., Molecular Formula | UV, IR Spectrum | NMR Spectrum ($d_6$-Acetone) |
|---|---|---|---|---|
| 15 | 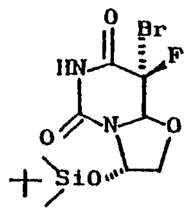 | mp 152-153°C<br>$C_{12}H_{20}BrFN_2O_4Si$ (383.302)<br>Anal. C H N F<br>cal. 25.13 3.52 4.89 3.31<br>F 25.37 3.21 5.03 3.22 | IR(KBr) 1756, 1692 $cm^{-1}$ | ($CD_3Cl$) 0.13(3H,S), 0.22(3H,S), 0.92(9H,S), 4.03(1H,dd,J=1&8Hz), 4.28(1H,dd,J=4&8Hz), 5.60(1H,d, J=18Hz), 5.98(1H,dd,J=1&4Hz), 8.4(1H,br) |
| 16 | 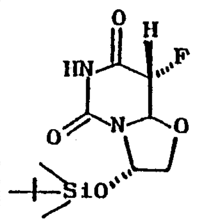 | mp 165-166°C<br>$C_{12}H_{21}FN_2O_4Si$ (304.396)<br>Anal. C H N F<br>cal. 47.35 6.95 9.20 6.24<br>F 47.45 7.18 9.14 6.51 | IR(KBr) 1752, 1715(sh) 1688 $cm^{-1}$ | 0.16(3H,S), 0.22(3H,S), 0.90(9H, S), 3.90(1H,dd,J=1.5&9Hz), 4.32 (1H,dd,J=4.5&9Hz), 5.07(1H,dd, J=8.5&50.3Hz), 5.60(1H,dd,J= 8.5&10Hz), 5.87(1H,m) |
| 17 | 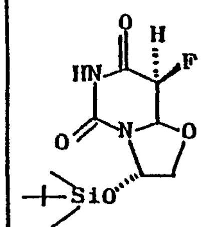 | mp 132-133°C<br>$C_{12}H_{21}FN_2O_4Si$ (304.396)<br>Anal. C H N F<br>cal. 47.35 6.95 9.20 6.24<br>F 47.52 7.18 9.13 6.23 | IR(KBr) 1736, 1698 $cm^{-1}$ | 0.13(3H,S), 0.23(3H,S), 0.92(9H, S), 3.95(1H,dd,J=8&1Hz), 4.25 (1H,dd,J=8&4Hz), 5.00(1H,dd,J= 50&2Hz), 5.70(1H,dd,J=24&2Hz), 5.98(1H,dd,J=1&4Hz), 9.67(1H, br) |
| 18 | 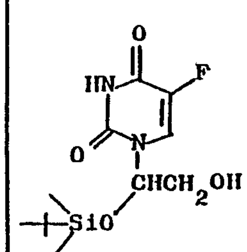 | mp 176-179°C<br>$C_{12}H_{21}FN_2O_4Si$ (304.396)<br>Anal. C H N F<br>cal. 47.35 6.95 9.20 6.24<br>F 47.19 7.04 9.02 6.51 | UV: $\lambda_{max}^{EtOH}$ 269mμ (ε=8,770)<br>IR(KBr) 3400, 1710(br), 1660 $cm^{-1}$ | 0.10(3H,S), 0.17(3H,S), 0.92(9H, S), 3.73(2H,d,J=6Hz), 6.03(1H, dt,J=2&6Hz), 7.80(1H,d,J=7Hz) |

| Compd. No | Structural Formula | m.p., Molecular Formula | UV, IR Spectrum | NMR Spectrum ($d_6$-Acetone) |
|---|---|---|---|---|
| 19 | 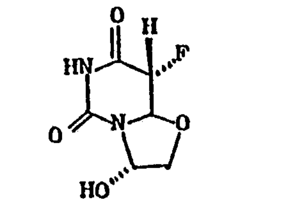 | mp 153-158°C<br>$C_6H_7FN_2O_4$ (190.136)<br>Anal. C H N F<br>cal. 37.90 3.71 14.74 9.99<br>F 37.79 3.70 14.68 10.33 | IR(KBr) 3470, 1735, 1700 $cm^{-1}$ | 3.85(1H,dd,J=3&9.5Hz), 4.37(1H,dd,J=5&9.5Hz), 5.08(1H,dd,J=8.5&49Hz), 558(1H,dd,J=8.5&11Hz), 5.80(1H,m) |
| 20 | 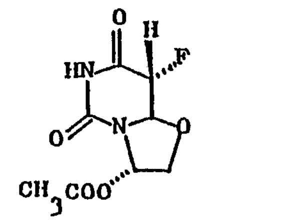 | mp 182-185°C<br>$C_8H_9FN_2O_5$ (232.172)<br>Anal. C H N F<br>cal. 41.39 3.91 12.07 8.18<br>F 41.12 4.12 11.07 8.15 | IR(Nujol) 1755 (sh), 1740, 1700 $cm^{-1}$ | 2.07(3H,S), 2.79(1H,br), 4.09(1H,dd,J=2&10Hz), 4.47(1H,dd,J=4.5&10Hz), 5.30(1H,dd,J=8.5&48.8Hz), 5.59(1H,dd,J=8.5&12Hz), 6.58(1H,ddd,J=2,3&4.5Hz) |
| 21 | 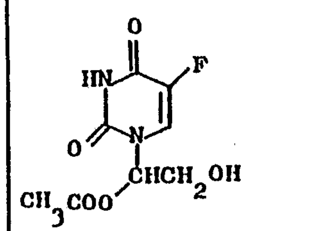 | mp 280-285°C<br>$C_8H_9FN_2O_5$ (232.172)<br>Anal. C H N F<br>cal. 41.38 3.91 12.07 8.18<br>F 41.44 3.99 11.93 8.10 | VV:$\lambda_{max}^{EtOH}$ 266mμ (ε=8,300)<br>IR(Nujol) 3500, 3200-3500, 1755, 1705, 1663 $cm^{-1}$ | 2.13(3H,S), 3.87(2H,d,J=5Hz), 6.77(1H,dt,J=2&5Hz), 7.85(1H,d,J=7Hz) |
| 22 | 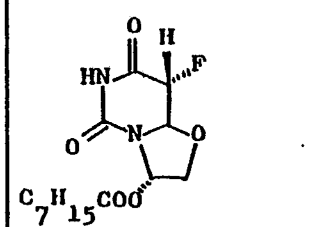 | mp 138-141°C<br>$C_{14}H_{21}FN_2O_5$ (316.33)<br>Anal. C H N F<br>cal. 53.15 6.69 8.86 6.01<br>F 52.51 6.51 8.82 5.96 | IR($CHCl_3$) 3370, 1735 $cm^{-1}$ | 0.87(3H,t,J=5Hz), 1.0-2.0(10H,m), 2.35(3H,t,J=6Hz), 4.01(1H,dd,J=2&9Hz), 4.50(1H,dd,J=4&9Hz), 5.18(1H,dd,J=8.5&50.4Hz), 5.68(1H,dd,J=8.5&9.6Hz), 6.58(1H,m) |

| Compd No. | Structural Formula | m.p., Molecular Formula | UV, IR Spectrum | MNR Spectrum ($d_6$-Acetone) |
|---|---|---|---|---|
| 23 | 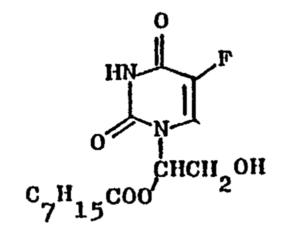 | mp 107-111°C<br>$C_{14}H_{21}FN_2O_5$ (316.33)<br>Anal. C H N F<br>cal. 53.16 6.69 8.86 6.01<br>F 52.88 6.65 8.78 5.77 | VV: $\lambda_{max}^{EtOH}$ 266.5 mμ(ε=8,500)<br>IR(CHCl₃) 3360, 3600-3300, 1757, 1710, 1670 $cm^{-1}$ | 0.88(3H,t,J=5Hz), 1.1-1.9(10H, m), 2.45(2H,t,J=7Hz), 3.93(2H, d,J=5Hz), 6.80(1H,dt,J=1&5Hz), 7.85(1H,d,J=7Hz) |
| 24 | 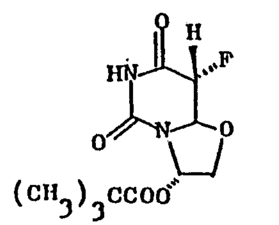 | mp 164-166°C<br>$C_{11}H_{15}FN_2O_5$ (274.25)<br>Anal. C H N F<br>cal. 48.17 5.51 10.22 6.93<br>F 47.43 5.30 10.08 6.70 | IR($CHCl_3$) 3380, 1740 $cm^{-1}$ | 1.20(9H,S), 4.00(1H,dd,J=2&10 Hz), 4.52(1H,dd,J=5&10Hz), 5.18 (1H,dd,J=8&51.5Hz), 5.67(1H,dd, J=8&8.5Hz), 6.57(1H,m) |
| 25 | 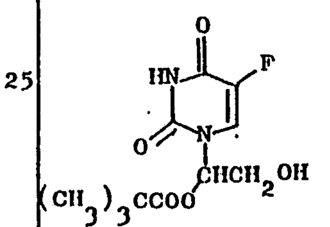 | mp 151-152°C<br>$C_{11}H_{15}FN_2O_5$ (274.25)<br>Anal. C H N F<br>cal. 48.17 5.51 10.22 6.93<br>F 47.83 5.43 10.21 7.02 | VV: $\lambda_{max}^{MeOH}$ 266mμ (ε=8,500)<br>IR($CHCl_3$) 3380, 3550-3300, 1750, 1715 $cm^{-1}$ | 1.22(9H,S), 3.90(2H,d,J=5Hz), 6.73(1H,dt,J=2&5Hz), 7.82(1H, d,J=7Hz), |
| 26 | 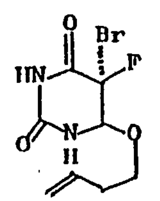 | mp 136.5-139°C<br>$C_8H_{10}BrFN_2O_3$ (281.096)<br>Anal. C H N F<br>cal. 34.18 3.59 9.97 6.76<br>F 33.86 3.49 9.88 6.91 | IR(Nujol) 1756, 1704 $cm^{-1}$ | 2.33(2H,m), 3.83(2H,m), 5.4-5.9(3H,m), 5.5-6.2(1H,m), 8.25 (1H,br) |

| Compd. No. | Structural Formula | m.p., Molecular Formula | UV, IR Spectrum | NMR Spectrum ($d_6$-Acetone) |
|---|---|---|---|---|
| 27 | 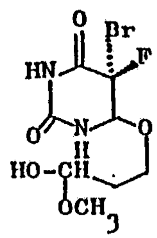 | mp 99-102°C<br>$C_8H_{12}BrFN_2O_5$ (315.112)<br>Anal. C H N F<br>cal. 30.49 3.84 8.89 6.03<br>F 30.24 3.60 9.02 6.16 | IR($CHCl_3$) 3380, 3100-3500, 1740 $cm^{-1}$ | 1.53-2.1(2H,m), 3.33(3H,S), 3.7-4.2(2H,m), 4.40(1H,t,J=6 Hz), 5.33(1H,dd,J=6&10Hz), 8.42 (1H,br), 9.92(1H br) |
| 28 | 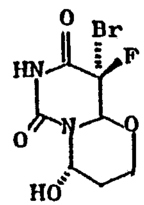 | mp 137.5-139.5°C<br>$C_7H_8BrFN_2O_4$ (283.07)<br>Anal. C H N F<br>cal. 29.70 2.85 9.90 6.71<br>F 29.79 2.86 9.62 6.81 | IR(Nujol) 3400, 3100-3500, 1740, 1710 $cm^{-1}$ | 1.5-2.5(2H,m), 3.8-4.6(2H,m), 5.7(1H,S), 5.97(1H,m), 9.3-11.0(1H,br) |
| 29 | 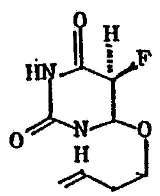 | mp 167-169°C<br>$C_8H_{11}FN_2O_3$ (202.188)<br>Anal. C H N F<br>cal. 47.52 5.48 13.86 9.40<br>F 47.17 5.33 13.76 9.31 | IR(Nujol) 1750, 1720, 1700 $cm^{-1}$ | 2.30(2H,m), 3.3-4.1(2H,m), 4.8-5.4(3H,m), 5.5-6.2(2H,m), 8.03 (1H,br), 9.87(1H,br) |
| 30 | 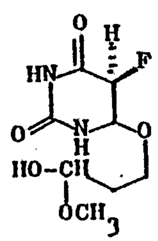 | $C_8H_{13}FN_2O_5$ (236.206) | IR(Nujol) 3100-3500, 1742, 1715, 1685 $cm^{-1}$ | |

| Compd. No. | Structural Formula | m.p., Molecular Formula | UV, IR Spectrum | MNR Spectrum ($d_6$-Acetone) |
|---|---|---|---|---|
| 31 | 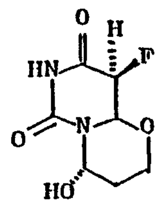 | mp 126-128°C<br>$C_7H_9FN_2O_4$ (204.162)<br>Anal. C H N F<br>cal. 41.18 4.44 13.72 9.31<br>F 41.42 4.17 13.80 9.41 | IR(Nujol) 3480, 1740, 1690 $cm^{-1}$ | 1.5-2.4(2H,m), 3.7-4.5(2H,m), 5.27(1H,dd,J=4&36Hz), 5.70(1H, dd,J=4&6Hz), 5.95(1H,m) |
| 32 | 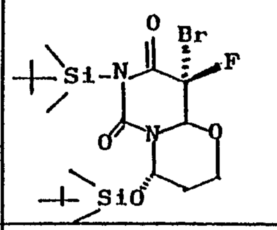 | mp 152-155°C<br>$C_{19}H_{36}BrFN_2O_4Si_2$(511.588)<br>Anal. C H N F<br>cal. 44.60 7.09 5.48 3.71<br>F 44.82 7.31 5.29 3.98 | IR($CHCl_3$) 3380, 1760, 1710 $cm^{-1}$ | 0.03(6H,S), 0.22(6H,S), 0.90 (9H,S), 0.98(9H,S), 1.4-2.4 (2H,m), 3.8-4.6(2H,m), 5.67 (1H,S), 6.07(1H,m) |
| 33 | 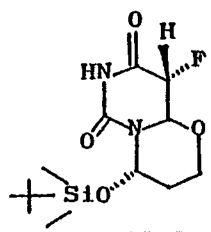 | mp 134-137°C<br>$C_{13}H_{23}FN_2O_4Si$ (318.422)<br>Anal. C H N F<br>cal. 49.03 7.28 8.80 5.97<br>F 49.02 7.53 8.71 6.31 | IR($CHCl_3$) 3380, 1750, 1710 $cm^{-1}$ | 0.17(6H,S), 0.95(9H,S), 1.4-2.0(2H,m), 3.7-4.7(2H,m),5.18 (1H,dd,J=4&29Hz), 5.58(1H.dd,J =4&12Hz), 5.92(1H,m) |
| 34 | 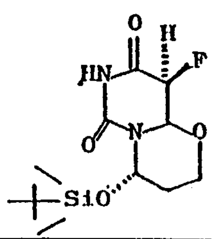 | mp 122-125°C<br>$C_{13}H_{23}FN_2O_4Si$ (318.422)<br>Anal. C H N F<br>cal. 49.03 7.28 8.80 5.97<br>F 49.23 7.52 8.75 5.84 | IR($CHCl_3$) 3380, 1755, 1710 $cm^{-1}$ | 0.10(3H,S), 0.17(3H,S), 0.90 (9H,S), 1.5-2.0(2H m), 3.8-4.4(2H,m), 4.95(1H,dd,J=7&30 Hz), 5.45(1H,dd,J=7&9Hz), 5.95 (1H,m) |

| Compd. No. | Structural Formula | m.p., Molecular Formula | UV, IR Spectrum | NMR Spectrum ($d_6$-Acetone) |
|---|---|---|---|---|
| 35 | 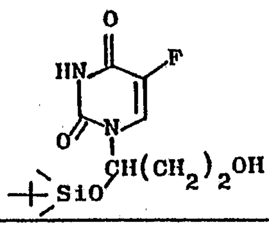 | mp 131-133°C<br>$C_{13}H_{23}FN_2O_4Si$ (318.422)<br>Anal. C H N F<br>cal. 49.03 7.28 8.80 5.97<br>F 49.15 7.47 8.65 5.78 | VV: $\lambda^{EtOH}_{max}$ 269mμ<br>(ε=8,700)<br>IR($CHCl_3$) 3390, 1705, 1675 $cm^{-1}$ | 0.08(3H S), 0.20(3H,S), 0.92 (9H,S), 1.8-2.2(2H,m), 3.67 (2H,d,J=6Hz), 6.23(1H,dt,J=2& 6Hz), 7.77(1H,d,J=6Hz) |
| 36 | 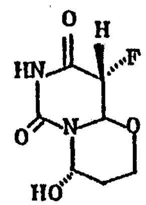 | mp 148-152°C<br>$C_7H_9FN_2O_4$ (204.162)<br>Anal. C H N F<br>cal. 41.18 4.44 13.72 9.31<br>F 41.44 4.35 13.38 9.51 | IR(Nujol) 3100-3500, 1760, 1732, 1715, 1690 $cm^{-1}$ | 1.6-2.1(2H,m), 3.8-4.4(2H,m), 4.97(1H,dd,J=8&30Hz), 5.45 (1H,dd,J=8&9Hz), 5.85(1H,m) |
| 37 | 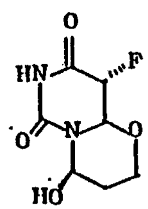 | mp 232-234°C'<br>$C_7H_9FN_2O_4$ (204.162)<br>Anal. C H N F<br>cal. 41.18 4.44 13.72 9.31<br>F 40.75 4.09 13.93 9.39 | IR(Nujol) 3100-3500, 1765, 1750, 1715, 1682 $cm^{-1}$ | 1.7-2.2(2H,m), 3.8-4.2(2H,m), 4.97(1H,dd,J=8&30Hz), 5.47 (1H,dd,J=8&9Hz), 5.85(1H,m) |
| 38 | 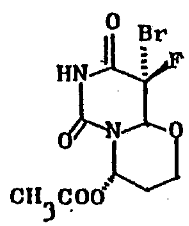 | mp 171-174°C<br>$C_9H_{10}BrFN_2O_5$ (325.106)<br>Anal. C H N F<br>cal. 33.25 3.10 8.62 5.84<br>F 33.19 3.12 8.44 6.13 | IR($CHCl_3$) 3380, 1760, 1710 $cm^{-1}$ | 1.83-2.57(2H,m), 2.17(1H,S), 4.1-5.6(2H,m), 5.70(1H,d,J= 2Hz), 6.83(1H,m) |

| Compd. No. | Structural Formula | m.p., Molecular Formula | UV, IR Spectrum | NMR Spectrum ($d_6$-Acetone) |
|---|---|---|---|---|
| 39 | 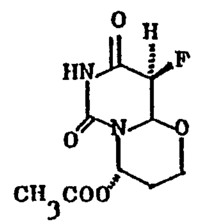 | mp 150-154°C<br>$C_9H_{11}FN_2O_5$ (246.198)<br>Anal. C H N F<br>cal. 43.91 4.50 11.38 7.72<br>F 44.15 4.48 11.35 7.95 | IR(Nujol) 1760, 1730, 1710 $cm^{-1}$ | 2.03(3H,S), 1.7-2.3(2H,m), 3.9-4.3(2H,m), 5.03(1H,dd,J=8&24Hz), 5.53(1H,dd,J=8&12Hz), 6.77(1H,m) |
| 40 | 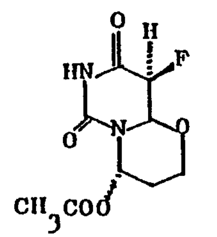 | mp 153.5-155.5°C<br>$C_9H_{11}FN_2O_5$ (246.198)<br>Anal. C H N F<br>cal. 43.91 4.50 11.38 7.72<br>F 44.19 4.14 11.43 7.90 | IR(Nujol) 1760, 1735 $cm^{-1}$ | 2.10(3H,S), 1.8-2.4(2H,m), 4.0-4.3(2H,m), 5.23(1H,dd,J=4&32Hz), 5.67(1H,dd,J=4&8Hz), 6.78(1H,m) |
| 41 | 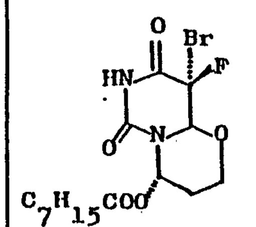 | mp 107-110°C<br>$C_{15}H_{22}BrFN_2O_5$ (409.262)<br>Anal. C H N F<br>cal. 44.02 5.42 6.85 4.64<br>F 44.14 5.45 6.70 4.58 | IR($CHCl_3$) 3380, 1760, 1710 $cm^{-1}$ | 0.88(3H,t,J=5Hz), 1.1-2.4(2H and 10H,m), 2.50(2H,t,J=7Hz), 4.0-4.6(2H,m), 5.63(1H,d,J=2Hz), 6.83(1H,m) |
| 42 | 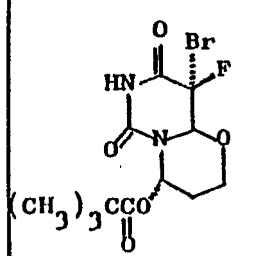 | mp 178-179.5°C<br>$C_{12}H_{16}BrFN_2O_5$ (367.182)<br>Anal. C H N F<br>cal. 39.25 4.39 7.63 5.17<br>F 39.77 4.41 7.35 4.95 | IR(Nujol) 1760, 1745, 1690 $cm^{-1}$ | 1.25(9H,S), 1.8-2.4(2H,m), 4.0-4.5(2H,m), 5.58(1H,d,J=2Hz), 6.80(1H,m) |

| Compd. No. | Structural Formula | m.p., Molecular Formula | UV, IR Spectrum | NMR Spectrum ($d_6$-Acetone) |
|---|---|---|---|---|
| 43 | 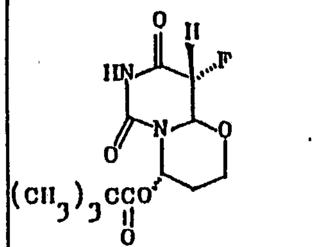 | mp 163-166°C<br>$C_{12}H_{17}FN_2O_5$ (288.276)<br>Anal. C H N F<br>cal. 49.99 5.95 9.72 6.59<br>F 49.78 5.85 9.58 6.67 | IR(Nujol) 1760, 1730, 1700 cm-1 | 1.20(9H,S), 1.7-2.6(2H,m), 3.9-4.3(2H,m), 5.08(1H,dd,J=8&34Hz), 5.58(1H,dd,J=8&8.5 Hz), 6.77(1H m) |
| 44 | 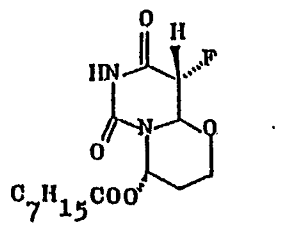 | mp 85-86°C<br>$C_{15}H_{23}FN_2O_5$ (330.356)<br>Anal. C H N F<br>cal. 54.53 6.99 8.48 5.75<br>F 54.45 6.94 8.45 5.78 | IR(CHCl$_3$) 1740, 3380 cm-1 | 0.87(3H,t,J=5Hz), 1.1-2.4(2H& 10H,m), 2.37(2H,t,J=7Hz), 3.9-4.4(2H,m), 5.07(1H,dd,J=8&29.5 Hz), 5.55(1H,dd,J=8&12Hz), 6.85(1H,m) |
| 45 | 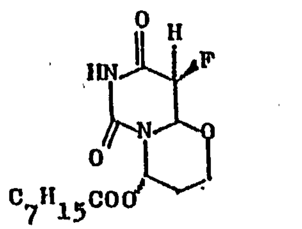 | oil<br>$C_{15}H_{23}FN_2O_5$ (330.356) | IR(CHCl$_3$) 3380, 1750, 1710 cm-1 | 0.88(3H,t,J=5Hz), 1.1-2.4(2H& 10H,m), 2.42(2H,t,J=6Hz), 3.9-4.3(2H,m), 5.17(1H,dd,J=4& 32Hz), 5.63(1H,dd,J=4&8Hz), 6.75(1H,m) |

## CLAIMS

1. A compound of the following general formula

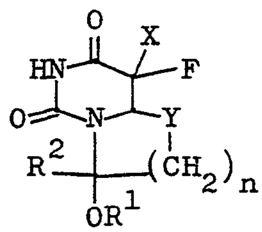

[wherein $R^1$ is hydrogen, $C_1$-$C_5$alkyl, $C_6$-$C_{10}$aryl, $C_7$-$C_{10}$ aralkyl, $C_1$-$C_{12}$alkanoyl, $C_2$-$C_6$alkoxycarbonyl, $C_1$-$C_5$alkanoyloxymethyl, carbamoyl of tri-$C_1$-$C_5$alkylsilyl ;

$R^2$ is hydrogen, $C_1$-$C_5$alkyl, $C_6$-$C_{10}$aryl or $C_7$-$C_{10}$ aralkyl ;

X is hydrogen, halogen or $C_2$-$C_6$alkoxycarbonyl ;

Y is O, NR' (R' is hydrogen or $C_1$-$C_5$alkyl), S, SO or $SO_2$ ; and

n is an integer of 1-3].

2. A compound claimed in claim 1, namely dl-8α-fluoro-2,3,6,7,8,8aα-hexahydro-5,7-dioxo-3α-trimethylacetoxy-5H-oxazolo[3,2-c]pyrimidine.

3. A compound claimed in claim 1, namely dl-8α-fluoro-2,3,6,7,8,8aα-hexahydro-3α-octanoyloxy-5,7-dioxo-5H-oxazolo[3,2-c]pyrimidine.

4. A compound claimed in claim 1, namely dl-3α-acetoxy-8α-fluoro-2,3,6,7,8,8aα-hexahydro-5,7-dioxo-5H-oxazolo[3,2-c]pyrimidine.

5. A compound claimed in claim 1, namely dl-9α-fluoro-3,4,7,8,9,9aα-hexahydro-6,8-dioxo-4α-trimethylacetoxy-2H,6H-[1,3]-oxazino[3,2-c]pyrimidine.

0064033

European Patent Office

# EUROPEAN SEARCH REPORT

Application number

EP 82 81 0175

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-2 017 082 (ELKAWI)<br>*Claim 1*<br>----- | 1 | C 07 D 498/04<br>C 07 D 513/04<br>C 07 D 487/04<br>C 07 F 7/18<br>A 61 K 31/55<br>A 61 K 31/505<br>A 61 K 31/695//<br>(C 07 D 498/04<br>C 07 D 263/00<br>C 07 D 239/00)<br>(C 07 D 498/04<br>C 07 D 265/00<br>C 07 D 239/00) |
| | | | TECHNICAL FIELDS SEARCHED (Int. Cl. 3) |
| | | | C 07 D 498/00<br>C 07 F 7/00<br>A 61 K 31/00 |

The present search report has been drawn up for all claims

| Place of search | Date of completion of the search | Examiner |
|---|---|---|
| THE HAGUE | 21-06-1982 | ALFARO I. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82